# EUROPEAN PATENT APPLICATION

(11) **EP 4 180 512 A1**
(43) Date of publication of application: **17.05.2023**
(21) Application number: 21841662.6
(22) Date of filing: 07.07.2021
(51) Int. Cl.: C12M 1/00, C12M 3/00, C12N 5/07, C12N 5/071, C12N 5/10

(54) **CELL CULTURING CONTAINER, METHOD FOR MANUFACTURING CELL CULTURING CONTAINER, CELL PRODUCTION METHOD, CELL CULTURING DEVICE, AND CELL CULTURING JIG**

(30) Priority: 11.07.2020 JP 2020119576; 11.07.2020 JP 2020119577; 31.07.2020 JP 2020130500; 16.06.2021 JP 2021099861
(71) Applicant: Toyo Seikan Group Holdings, Ltd., Shinagawa-ku Tokyo 141-8627 (JP)
(72) Inventor: KOSEKI, Osamu, Yokohama-shi, Kanagawa 240-0062 (JP); TANAKA, Satoshi, Yokohama-shi, Kanagawa 240-0062 (JP); TOTANI, Takahiko, Yokohama-shi, Kanagawa 240-0062 (JP); MATSUOKA, Yosuke, Tokyo 141-8627 (JP); NISHIYAMA, Takaharu, Yokohama-shi, Kanagawa 240-0062 (JP)
(74) Representative: Hasegawa, Kan
(86) International application number: PCT/JP2021/025607
(87) International publication number: WO 2022/014436

(57) **Abstract**

Provided is a cell culture vessel for culturing cells with a high density having excellent gas permeability and strength. A bag-shaped cell culture vessel 1 of a closed system includes at least one port and mutually opposed planar substrates 11. At least one of the planar substrates 11 is formed of a gas permeable film, the at least one gas permeable film has an outer surface provided with a protruding portion 111 having a shape different from an inner surface shape of the gas permeable film, a culture space S for culturing cells is provided in the inner surface side of the gas permeable film, and when the gas permeable film is brought in contact with a planar surface, a space enabling ventilation is formed between the gas permeable film and the planar surface by the protruding portion 111.

## Description

### TECHNICAL FIELD

The present invention relates to a cell culture technique, and especially relates to a cell culture vessel and a cell culture jig for culturing cells with a high density.

### BACKGROUND ART

Recently, in the production of pharmaceutical medicines and in the fields of gene therapy, regeneration medicine, immunotherapy, and the like, it is desired that cells and tissues are efficiently cultured in a large amount under an artificial environment.

In such a situation, it has been proposed that cells are automatically cultured in large quantities in a closed system using a bag-shaped cell culture vessel.

When cells are cultured using a bag-shaped cell culture vessel, the vessel is used in a manner of being placed in an incubator, or the vessel is used in a state of being sandwiched by a pressing jig for stabilizing a culture environment by suppressing a move of a culture fluid in the vessel in some cases.

However, there has been a problem that since the bag-shaped cell culture vessel is usually formed by bonding two films together, and its outer surface is flat, placing the vessel in the incubator possibly causes insufficient gas permeation through a bottom surface of the vessel.

That is, a hole punched plate referred to as a perforated metal is usually used for a placement table of the incubator, and its aperture rate is often 50% or less. Therefore, a surface of the cell culture vessel is in close contact with the placement table and the gas permeation into the vessel is interfered excluding the perforated part. When oxygen consumption by cells increases during the culture with a high density, the oxygen concentration around cells in the vessel falls down. This has been a problem.

The use in the state of being sandwiched by the pressing jig brings a base or a pressing member of the pressing jig in close contact with the vessel surface. Therefore, the gas permeation performance of the vessel is inhibited also in this case, and the oxygen concentration around cells is reduced, thus reducing cell proliferation efficiency. This has been a problem.

In mass culture of cells using a bag-shaped cell culture vessel, the vessel strength and the gas permeability magnitude are important.

For example, when a cell culture vessel is produced using a gas permeable film of polyethylene, ethylene vinyl acetate, or the like, a thickness of 100 µm or more has been usually required for ensuring the vessel strength.

However, in this case, when the density of cultured cells becomes a high density of 500,000 cells/cm² or more, the gas permeation performance of the vessel becomes insufficient, and the oxygen concentration around cells is reduced, thus reducing the proliferation efficiency. This has been a problem.

On the other hand, for example, when a cell culture vessel is produced using a gas permeable film having a thickness of 50 µm or less, while the gas permeation performance is sufficient even in the high-density state of the cultured cells, the vessel strength becomes insufficient and the bag is possibly broken, thus causing a difficulty in handling. This has been a problem.

### CITATION LIST

### Patent Literature

Patent Document 1: Japanese Patent No. 4806761
Patent Document 2: JP-A-2016-77164

### DISCLOSURE OF THE INVENTION

### PROBLEMS TO BE SOLVED BY THE INVENTION

Here, Patent Document 1 discloses that the gas permeation performance of a bag-shaped vessel is improved by providing a plurality of ventholes at positions of a holding plate of a culture tray at which the holding plate is in contact with the bag-shaped vessel.

However, since the cells usually accumulate in a lower surface side in the bag-shaped vessel, simply providing the ventholes to the holding plate provides a small effect of improving the gas permeation performance. Thus, the culture tray fails to sufficiently solve the problem of the reduced oxygen concentration around cells in the vessel.

Therefore, the inventors have seriously studied and succeeded in providing a sufficient gas permeation performance of a cell culture vessel even in a culture with a high density by providing a protruding portion on an outer surface of a gas permeable film constituting the vessel and forming a space enabling ventilation between the gas permeable film and a planar surface by the protruding portion when the gas permeable film is brought in contact with the planar surface.

With the cell culture vessel, the strength in handling can be ensured even when the thickness of the gas permeable film is less than 100 µm.

The inventors also succeeded in suppressing the inhibition of the gas permeation performance of a bag-shaped cell culture vessel in a culture by forming at least a stage with a substrate enabling ventilation between a surface of the cell culture vessel and the stage in a cell culture jig including the stage on which the cell culture vessel is placed and a pressing member that presses the cell culture vessel to the stage.

Incidentally, for improving the gas permeation performance and the strength of the bag-shaped cell culture vessel, a multilayer film formed by laminating films mutually different in material or density is used as a film constituting the vessel in some cases. However, the performance has not been significantly improved yet.

As a material excellent in gas permeation performance, a silicone material is included. While the silicone material is low in strength, the high gas permeation performance can be provided even with the thickness of 300 µm or more, and therefore, the strength in handling can be ensured.

However, there is a problem that welding a port or the like to the silicone material is difficult. Additionally, since harmful substances are released by radiation sterilization or the like, the silicone material possibly adversely affects the subsequent culture performance. Furthermore, since it is difficult to perform a surface treatment necessary for adherent cell culture to the silicone material, producing a culture vessel for adherent cells is difficult while producing a culture vessel for floating cells is possible. This has been a problem.

Therefore, the inventors formed a plurality of thin portions and projecting portions on a surface of a gas permeable film constituting a vessel, and made a thickness of the thin portion 75 µm or less, thereby succeeded in ensuring a strength in handling and providing a magnitude of gas permeability capable of culturing cells with a high density. Thus, the inventors completed the present invention.

Here, Patent Document 2 discloses a cell culture vessel including a support layer in a gas permeable film. However, the support layer is disposed to avoid a deflection of the gas permeable film placed to be flat in a vessel such as a flask, and the support layer is not configured to enhance the strength of the vessel. Thus, the cell culture vessel of Patent Document 2 is not capable of achieving the object of improving the gas permeation performance and the strength of the bag-shaped cell culture vessel and enabling the cell culture with a high density.

In contrast, with the above-described configuration, the strength in handling is ensured by the projecting portions formed on the surface of the gas permeable film constituting the vessel, and the gas permeation performance can be improved by the thin portion having a predetermined thickness.

The present invention has been made in view of the above circumstances and an object of the present invention is to provide a cell culture vessel excellent in gas permeability and strength for culturing cells with a high density, a method for producing the cell culture vessel, a method for producing cells, and a cell culture apparatus.

Additionally, an object of the present invention is to provide a cell culture jig capable of suppressing an inhibition of a gas permeation performance of the cell culture vessel and culturing cells with a high density.

### SOLUTIONS TO THE PROBLEMS

To achieve the above-described object, a cell culture vessel of the present invention is a bag-shaped cell culture vessel of a closed system. The cell culture vessel includes at least one port and mutually opposed planar substrates. At least one of the planar substrates is formed of a gas permeable film, the at least one gas permeable film has an outer surface provided with a protruding portion having a shape different from an inner surface shape of the gas permeable film, a culture space for culturing cells is provided in the inner surface side of the gas permeable film, and when the gas permeable film is brought in contact with a planar surface, a space enabling ventilation is formed between the gas permeable film and the planar surface by the protruding portion.

In the cell culture vessel of the present invention, it is preferred that in an outer surface region of the gas permeable film in an outer side of the culture space, a plurality of approximately triangular prisms are formed to be arranged in parallel in a mountain range pattern as the protruding portion.

Furthermore, in the cell culture vessel of the present invention, it is also preferred that both of the mutually opposed planar substrates are formed of a gas permeable film, and the protruding portions are formed on outer surfaces of the respective gas permeable films.

A method for producing a cell culture vessel of the present invention is a method for producing a bag-shaped cell culture vessel of a closed system. The cell culture vessel includes at least one port and mutually opposed planar substrates. The method includes: using a gas permeable film for at least one of the planar substrates, and forming a protruding portion having a shape different from an inner surface shape of the gas permeable film on an outer surface of the gas permeable film such that a space enabling ventilation is formed between the gas permeable film and a planar surface when the gas permeable film is brought in contact with the planar surface; and forming a culture space for culturing cells in the inner surface side of the gas permeable film by bonding peripheral edge portions of the mutually opposed planar substrates.

Furthermore, a method for producing cells of the present invention is a method for producing cells in which cells are cultured using the above-described cell culture vessel.

A cell culture apparatus of the present invention is a cell culture apparatus including the above-described cell culture vessel and a culture jig. The culture jig includes a flat stage on which the cell culture vessel is placed and a pressing member that presses the cell culture vessel placed on the stage. The culture jig is provided with a space enabling ventilation between the gas permeable film and the stage and/or between the gas permeable film and the pressing member by the protruding portion formed on the cell culture vessel.

To achieve the above-described object, a cell culture jig of the present invention is a cell culture jig for holding a bag-shaped cell culture vessel of a closed system, the cell culture vessel includes mutually opposed planar substrates, and at least one of the planar substrates is formed of a gas permeable film. The cell culture jig includes a stage on which the cell culture vessel is placed and a pressing member that presses the cell culture vessel to the stage. The stage is formed of a substrate enabling ventilation between the gas permeable film and the stage, and the gas permeable film side of the cell culture vessel is placed on the stage.

In the cell culture jig of the present invention, it is preferred that the pressing member is formed of a substrate enabling ventilation between the gas permeable film and the pressing member, and the gas permeable film side of the cell culture vessel is pressed by the pressing member.

In the cell culture jig of the present invention, it is preferred that guide pins are disposed upright on four corners of the stage, the guide pins pass through guide bores provided to the pressing member, and the pressing member is disposed to be movable in a perpendicular direction along the guide pins.

In the cell culture jig of the present invention, it is preferred that support pillars are disposed upright on four corners of the stage, a top panel is secured to the support pillars, the top panel includes guide pins, the guide pins pass through guide bores provided to the pressing member, and the pressing member is disposed to be movable in a perpendicular direction along the guide pins.

A method for producing cells of the present invention is a method for producing cells in which cells are cultured using the above-described cell culture jig.

Furthermore, to achieve the above-described object, a cell culture vessel of the present invention is a bag-shaped cell culture vessel of a closed system. The cell culture vessel includes at least one port and mutually opposed planar substrates. At least one of the planar substrates is formed of a gas permeable film, a culture space for culturing cells is provided in a vessel inner surface side of the gas permeable film, a plurality of thin portions and projecting portions are formed on the gas permeable film forming the culture space, and the thin portion has a thickness of 75 µm or less.

In the cell culture vessel of the present invention, it is also preferred that the thin portion has the thickness of 20 µm to 35 µm.

In the cell culture vessel of the present invention, it is also preferred that a surface of the gas permeable film on which the projecting portions are formed is a vessel outer surface of the gas permeable film, and a vessel inner surface of the gas permeable film is flat without unevenness.

Furthermore, in the cell culture vessel of the present invention, it is also preferred that a surface of the gas permeable film on which the projecting portions are formed is a vessel inner surface of the gas permeable film, a plurality of small protruding portions are formed on a vessel outer surface of the gas permeable film, and a space enabling ventilation is formed between the gas permeable film and a planar surface by the small protruding portions when the outer surface is brought in contact with the planar surface.

In the cell culture vessel of the present invention, it is also preferred that both of the mutually opposed planar substrates are formed of the gas permeable film, and the plurality of thin portions and the projecting portions are formed on each of the gas permeable films.

In the cell culture vessel of the present invention, it is also preferred that a plurality of approximately triangular prisms are formed to be arranged in parallel in a mountain range pattern as the projecting portions.

Furthermore, in the cell culture vessel of the present invention, it is also preferred that a plurality of approximately triangular prisms are formed to be arranged in parallel in a mountain range pattern as the projecting portions, a plurality of other approximately triangular prisms are formed to be arranged in parallel in a mountain range pattern so as to intersect with the plurality of approximately triangular prisms, and the plurality of approximately triangular prisms are provided in a grid pattern.

In the cell culture vessel of the present invention, it is also preferred that the projecting portions mutually adjacent in parallel are formed with intervals of 1 mm to 5 mm.

Furthermore, in the cell culture vessel of the present invention, it is also preferred that the projecting portions mutually adjacent in parallel are formed without intervals, and the thin portions are formed in line shapes or dot shapes.

A method for producing cells of the present invention is a method for producing cells in which cells are cultured using the above-described cell culture vessel.

A method for producing a cell culture vessel of the present invention is a method for producing a bag-shaped cell culture vessel of a closed system. The cell culture vessel includes at least one port and mutually opposed planar substrates. The method includes: using a gas permeable film for at least one of the planar substrates, and forming a plurality of thin portions having thicknesses of 75 µm or less and projecting portions on a surface of the gas permeable film; and forming a culture space for culturing cells in a vessel inner surface side of the gas permeable film by bonding peripheral edge portions of the mutually opposed planar substrates.

### EFFECTS OF THE INVENTION

The present invention can provide the cell culture vessel excellent in gas permeability and strength for culturing cells with a high density, the method for producing the cell culture vessel, the method for producing cells, and the cell culture apparatus.

Additionally, the present invention can provide the cell culture jig capable of suppressing the inhibition of the gas permeation performance of the cell culture vessel and culturing cells with a high density.

### BRIEF DESCRIPTION OF THE DRAWINGS

FIG. 1 is a drawing illustrating a plan view (a) and an A-A cross-sectional view (b) of a cell culture vessel according to a first embodiment of the present invention.
FIG. 2 is an enlarged view (a) of a gas permeable film used for the cell culture vessel according to the first embodiment of the present invention and a schematic diagram (b) illustrating a state of a cell culture by the vessel.
FIG. 3 is a drawing illustrating a state of performing the culture while securing the cell culture vessel according to the first embodiment of the present invention to a culture jig.
FIG. 4 is a drawing illustrating graphs indicating measurement results of a gas permeation performance of cell culture vessels (Example 1, Comparative Example 1) in Test 1.
FIG. 5 is a drawing illustrating a result of cell culture using cell culture vessels (Example 2, Comparative Example 2) in Test 2.
FIG. 6 is a perspective view (a) illustrating a cell culture jig and a cell culture vessel placed thereon according to a second embodiment of the present invention, and an enlarged view (b) of surfaces of a stage and a pressing member of the cell culture jig.
FIG. 7 is a front view illustrating the cell culture jig and the cell culture vessel placed thereon according to the second embodiment of the present invention.
FIG. 8 is a side view illustrating the cell culture jig and the cell culture vessel placed thereon according to the second embodiment of the present invention.
FIG. 9 is a schematic diagram illustrating a modification of the cell culture jig according to the second embodiment of the present invention and the cell culture vessel placed thereon.
FIG. 10 is a schematic diagram illustrating a state of a cell culture using the cell culture jig according to the second embodiment of the present invention.
FIG. 11 is a drawing illustrating a graph indicating a measurement result of a gas permeation performance of a cell culture vessel secured to a cell culture jig (Example 3, Comparative Example 3) in Test 3.
FIG. 12 is a drawing illustrating a graph indicating a measurement result of a gas permeation performance of a cell culture vessel secured to a cell culture jig (Example 4, Example 5) in Test 4.
FIG. 13 is a drawing illustrating a result of a cell culture using a cell culture jig (Example 6, Comparative Example 4) in Test 5.
FIG. 14 is a drawing illustrating a result of a cell culture using a cell culture jig (Example 7, Comparative Example 5) in Test 6.
FIG. 15 is a drawing illustrating a plan view (a) and an A-A cross-sectional view (b) of a cell culture vessel according to a third embodiment of the present invention.
FIG. 16 is an enlarged view (a) and a B-B cross-sectional view (b) of a gas permeable film used for the cell culture vessel according to the third embodiment of the present invention.
FIG. 17 is a drawing illustrating oxygen permeabilities of various kinds of gas permeable films used for the cell culture vessel according to the third embodiment of the present invention and the like.
FIG. 18 is a schematic diagram illustrating a state of performing a culture using the cell culture vessel according to the third embodiment of the present invention.
FIG. 19 is an enlarged view (a) and a C-C cross-sectional view (b) of a gas permeable film used for a cell culture vessel according to a fourth embodiment of the present invention.
FIG. 20 is a cross-sectional view (a) of a gas permeable film used for a cell culture vessel according to a fifth embodiment, and a cross-sectional view (b) of a gas permeable film used for a cell culture vessel according to a sixth embodiment of the present invention.
FIG. 21 is a cross-sectional view (a) of a gas permeable film used for a cell culture vessel according to a seventh embodiment, and a cross-sectional view (b) of a gas permeable film used for a cell culture vessel according to an eighth embodiment of the present invention.
FIG. 22 is a cross-sectional view (a) of a gas permeable film used for a cell culture vessel according to a ninth embodiment, and a cross-sectional view (b) of a gas permeable film used for a cell culture vessel according to a tenth embodiment of the present invention.

### DESCRIPTION OF EMBODIMENTS

The following describes embodiments of a cell culture vessel, a method for producing a cell culture vessel, a method for producing cells, and a cell culture apparatus of the present invention in detail. However, the present invention is not limited to the specific contents of the embodiments and examples below.

### [First Embodiment]

A cell culture vessel of the embodiment is a bag-shaped cell culture vessel of a closed system, and the cell culture vessel includes at least one port and mutually opposed planar substrates. At least one of the planar substrates is formed of a gas permeable film. The at least one gas permeable film has an outer surface provided with a protruding portion having a shape different from an inner surface shape of the gas permeable film. A culture space for culturing cells is provided in the inner surface side of the gas permeable film. When the gas permeable film is brought in contact with a planar surface, a space enabling ventilation is formed between the gas permeable film and the planar surface by the protruding portion.

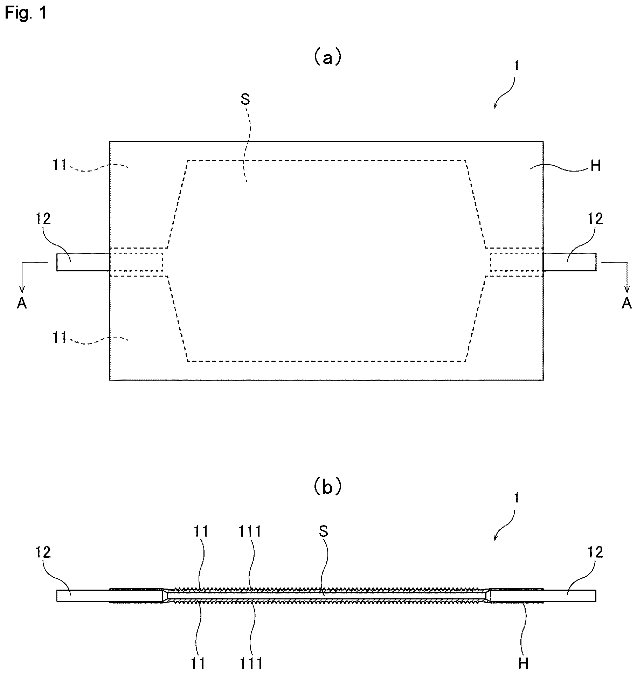

Specifically, as illustrated in FIG. 1(a), a cell culture vessel 1 of the embodiment can be obtained by, for example, thermally welding peripheral edge portions H of two rectangular planar substrates 11 by a heat seal. A space formed between the two planar substrates 11 is used as a culture space S for culturing cells. The regions forming the culture space S of the planar substrates 11 constitute a culture portion in the cell culture vessel 1. The entire shape of the planar substrate 11, the shape of the peripheral edge portion H, and the shape of the culture space S are not especially limited, and those having any shapes can be used.

At least one of the planar substrates 11 is formed of a gas permeable film, and it is preferred to use one in which both are formed of the gas permeable film.

The "planar" of the planar substrate 11 does not mean that the substrate surface is flat without unevenness, but means that the entire shape of the substrate is roughly flat, and includes one in which the unevenness is formed on the surface of the planar substrate 11.

As illustrated in FIG. 1(b), in the cell culture vessel 1 of the embodiment, a protruding portion 111 is provided to the outer surface of the planar substrate 11 as the gas permeable film, and when the gas permeable film is brought in contact with a planar surface, a space enabling ventilation is formed between the gas permeable film and the planar surface by the protruding portion 111.

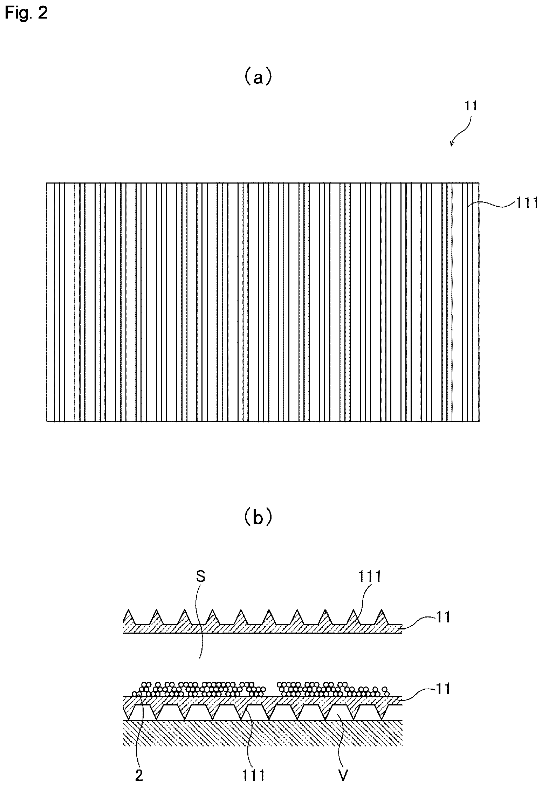

While the shape of the protruding portion 111 of the embodiment is not especially limited, for example, as illustrated in FIG. 1(b) and FIG. 2, the shape formed by arranging a plurality of approximately triangular prisms in parallel in a mountain range shape (hereinafter referred to as a mountain range pattern in some cases) is preferable.

By thus forming the protruding portion 111 in the mountain range pattern, a contacted area of the gas permeable film with the planar surface can be reduced, and the inhibition of the gas permeation performance of the cell culture vessel 1 can be avoided.

With the mountain range pattern, strengths of the protrusions of the protruding portion 111 can be enhanced, and for example, a damage such as a distortion of the protrusion due to the pressing force of the pressing member can be avoided.

Furthermore, the protruding portion 111 may be formed by arranging a plurality of cuboids in parallel, and other various shapes may be employed.

The protruding portion 111 of the embodiment is preferably formed in an outer surface region of the gas permeable film in the outer side of the culture space S.

It is also preferred that the gas permeable film is used for both of the mutually opposed planar substrates 11, and the protruding portions 111 are disposed on the outer surfaces of the respective gas permeable films.

By disposing the protruding portion 111 in the cell culture vessel 1 of the embodiment, when the cell culture vessel 1 is placed on a planar surface, a space enabling ventilation is formed between the gas permeable film facing the planar surface and the planar surface. Therefore, the gas permeation performance of the cell culture vessel 1 can be significantly improved.

When an upper surface of the cell culture vessel 1 is pressed by the pressing member, a space enabling ventilation is formed between the gas permeable film facing the planar surface of the pressing member and the planar surface. Therefore, the gas permeation performance of the cell culture vessel 1 can be significantly improved also in this case.

Furthermore, by sending air to the space between the gas permeable film and the planar surface formed by the protruding portion 111, the gas permeation performance of the cell culture vessel 1 can be more improved.

By thus forming the protruding portion 111 in the cell culture vessel 1 of the embodiment, the strength of the cell culture vessel 1 also can be enhanced.

The shape of the protruding portion 111 of the embodiment is preferably different from the inner surface shape of the gas permeable film, and is different from a shape, for example, obtained by forming a plurality of wells on an inner surface of a vessel and forming hemispherical shapes on an outer side of the vessel by outer surfaces of the wells.

That is, the protruding portion 111 formed on the outer surface of the gas permeable film in the cell culture vessel 1 of the embodiment preferably has a shape enabling more appropriate ventilation between the gas permeable film and the planar surface regardless of the inner surface shape of the gas permeable film.

Accordingly, the inner surface shape of the gas permeable film in the cell culture vessel 1 of the embodiment can be set regardless of the shape of the protruding portion 111, and a flat shape without unevenness is also preferable. It is also preferable that a culture area inside the vessel is increased by forming unevenness on the inner surface of the gas permeable film regardless of the shape of the protruding portion 111.

As the planar substrate 11, a resin film or the like can be preferably used, and for example, a polyolefin-based resin such as polyethylene and polypropylene is usable. For example, polyethylene, a copolymer of ethylene and α-olefin, a copolymer of ethylene and vinyl acetate, an ionomer using: a copolymer of ethylene and an acrylic acid or ethylene and a methacrylic acid; and a metal ion, and the like can be listed. Further, a polyolefin, a styrene-based elastomer, a polyester-based thermoplastic elastomer, a silicone-based thermoplastic elastomer, a silicone resin, and the like can be used. Furthermore, a silicone rubber, a soft vinyl chloride resin, a polybutadiene resin, an ethylene-vinyl acetate copolymer, a chlorinated polyethylene resin, a polyurethane-based thermoplastic elastomer, a polyester-based thermoplastic elastomer, a silicone-based thermoplastic elastomer, a styrene-based elastomer, such as styrene-butadiene-styrene (SBS), styrene-isoprene-styrene (SIS), styrene-ethylene-butylene-styrene (SEBS), and styrene-ethylene-propylene-styrene (SEPS), a polyolefin resin, a fluorine-based resin, and the like may be used.

As the gas permeable film, among the above-described materials of the planar substrate 11, especially, using a thermoplastic resin excellent in gas permeation performance is preferable, and a polyolefin-based resin, for example, polyethylene, such as linear low density polyethylene (LLDPE), or polypropylene can be preferably used. To make the inside of the cell culture vessel 1 visible, the gas permeable film is preferably a transparent material.

Here, for the gas permeable film, generally, the smaller the thickness is, the higher the gas permeation performance becomes, while the smaller the thickness of the gas permeable film is, the more difficult its handling becomes. Especially, since one having the thickness of 30 µm or 20 µm is like a food wrap (thickness about 10 µm), the vessel strength is insufficient, thus possibly causing a break.

However, since the cell culture vessel 1 of the embodiment includes the protruding portion on the outer surface of the gas permeable film, the cell culture vessel 1 can be handled even when the thickness of the gas permeable film is relatively thin.

In view of this, the thickness of the gas permeable film in the cell culture vessel 1 of the embodiment is preferably less than 100 µm, more preferably 70 µm or less and 10 µm or more, and further preferably 35 µm or less and 20 µm or more.

FIG. 2(b) illustrates a state of a cell culture by the cell culture vessel 1 of the embodiment.

The drawing illustrates a state of performing the culture in which a culture fluid is filled in the cell culture vessel 1, and cells 2 are seeded in the culture space S whose volume has been enlarged by the culture fluid. A ventilation space V is formed between the protruding portion 111 and a placement surface of the cell culture vessel 1.

Thus, the cell culture vessel 1 of the embodiment is excellent in gas permeation performance when performing the cell culture, and the cells can be cultured with a high density.

The cell culture vessel 1 of the embodiment includes at least one port 12 sandwiched between the peripheral edge portions H of the two planar substrates 11 and thermally welded.

While the two ports 12 are disposed to be mutually opposed at both ends in a longitudinal direction of the planar substrate 11 in FIG. 1, the number of the ports 12 is not limited thereto, and the number of the ports 12 may be one, or three or more.

As the material of the port 12, for example, a thermoplastic resin, such as polyethylene, polypropylene, vinyl chloride, polystyrene-based elastomer, or FEP can be used.

In a method for producing cells of the embodiment, cells are cultured using the above-described cell culture vessel 1.

The cells cultured using the cell culture vessel 1 are not especially limited, and the cells may be floating cells, such as lymphocytes or dendritic cells, cultured by floating cells in a culture fluid, or may be adherent cells, such as induced pluripotent stem cells (iPS cells), neural stem cells, embryonic stem cells (ES cells), mesenchymal stem cells, hepatic cells, islet cells, cardiac muscle cells, corneal endothelial cells, and lymph corpuscles in an activation process, cultured by making cells adhere to a culture portion in the vessel.

A method for producing a cell culture vessel of the embodiment is a method for producing a bag-shaped cell culture vessel of a closed system, and the cell culture vessel includes at least one port and mutually opposed planar substrates. A gas permeable film is used for at least one of the planar substrates. A protruding portion having a shape different from an inner surface shape of the gas permeable film is formed on an outer surface of the gas permeable film such that a space enabling ventilation is formed between the gas permeable film and a planar surface when the gas permeable film is brought in contact with the planar surface. A culture space for culturing cells is formed in the inner surface side of the gas permeable film by bonding peripheral edge portions of the mutually opposed planar substrates.

In the method for producing a cell culture vessel of the embodiment, it is also preferred that the gas permeable film is used for both of the mutually opposed planar substrates, and the protruding portions are formed on the outer surfaces of the respective gas permeable films.

Furthermore, the protruding portion is preferably formed by a thermal transfer, a cast film forming, or an inflation film forming.

According to the method for producing a cell culture vessel, a cell culture vessel excellent in gas permeation performance and strength can be produced.

A cell culture apparatus of the embodiment includes the above-described cell culture vessel and a culture jig. The culture jig includes a flat stage on which the cell culture vessel is placed and a pressing member that presses the cell culture vessel placed on the stage. The culture jig is provided with a space enabling ventilation between the gas permeable film and the stage and/or between the gas permeable film and the pressing member by the protruding portion formed on the cell culture vessel.

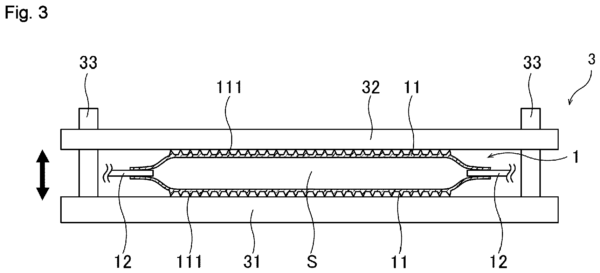

That is, as illustrated in FIG. 3, a cell culture apparatus 3 of the embodiment includes the cell culture vessel 1, a stage 31, a pressing member 32, and guide pins 33.

The cell culture vessel 1 is placed on the stage 31, and the pressing member 32 presses the upper surface of the cell culture vessel 1. The guide pins 33 are disposed upright on four corners of the stage 31, and the guide pins 33 pass through guide bores provided to the pressing member 32. Then, the pressing member 32 is supported to be movable in a perpendicular direction along these guide pins 33.

At this time, the pressing member 32 can be fixed by the own weight so as to press the cell culture vessel 1 with a constant pressure. Spring materials may be installed to the guide pins 33 to appropriately press the pressing member 32 to the cell culture vessel 1.

With the configuration of the cell culture apparatus 3 of the embodiment, the pressing member 32 can be configured to be movable in the perpendicular direction in accordance with injection/discharge of the culture fluid to/from the cell culture vessel 1.

In the cell culture apparatus 3 of the embodiment, since the cell culture vessel 1 includes the protruding portion 111, a space enabling ventilation is formed between the gas permeable film facing the upper surface of the stage 31 and the upper surface of the stage 31. Additionally, a space enabling ventilation is formed between the gas permeable film facing the lower surface of the pressing member 32 and the lower surface of the pressing member 32.

Therefore, the cell culture apparatus 3 of the embodiment can suppress the inhibition of the gas permeation performance of the cell culture vessel 1 caused by sandwiching the cell culture vessel 1 between the stage 31 and the pressing member 32.

Next, embodiments of the cell culture jig and the method for producing cells of the present invention will be described in detail. However, the present invention is not limited to the specific contents of the embodiments and examples below.

### [Second Embodiment]

The cell culture jig of the embodiment is a cell culture jig for holding a bag-shaped cell culture vessel of a closed system, and the cell culture vessel includes mutually opposed planar substrates at least one of which is formed of a gas permeable film. The cell culture jig includes a stage on which the cell culture vessel is placed and a pressing member that presses the cell culture vessel to the stage. The stage is formed of a substrate enabling ventilation between the gas permeable film and the stage, and the gas permeable film side of the cell culture vessel is placed on the stage.

It is preferred that in the cell culture jig of the embodiment, the pressing member is formed of a substrate enabling ventilation between the gas permeable film and the pressing member, and the gas permeable film side of the cell culture vessel is pressed by the pressing member.

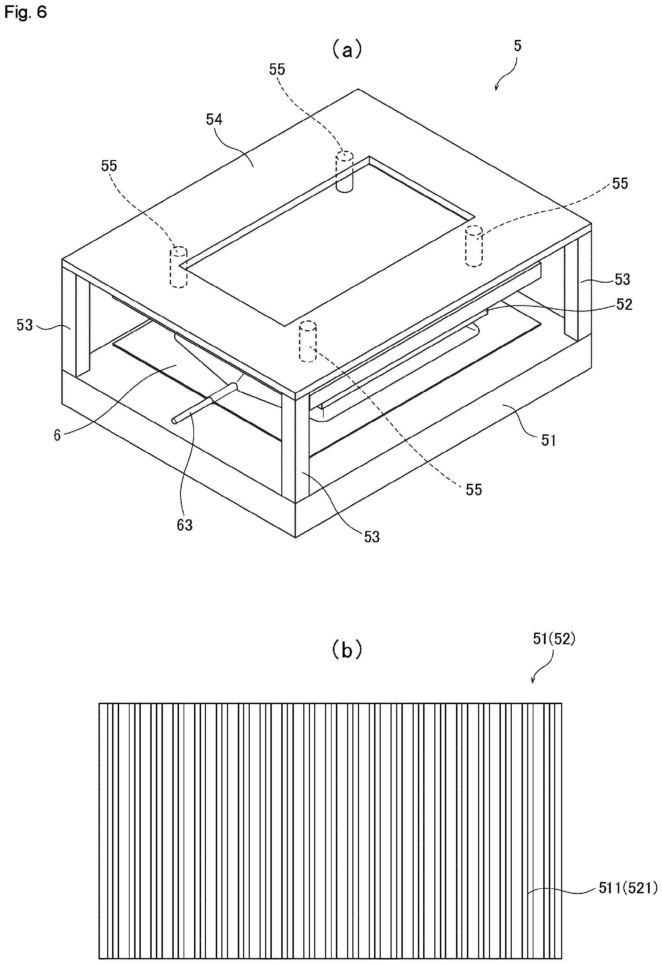

Specifically, as illustrated in FIG. 6 to FIG. 8, a cell culture jig 5 of the embodiment includes a stage 51 on which a cell culture vessel 6 is placed and a pressing member 52 that presses the cell culture vessel 6 to the stage 51.

Support pillars 53 are disposed upright on four corners of the stage 51, and a top panel 54 is secured to the support pillars 53. The top panel 54 includes guide pins 55, the guide pins 55 pass through guide bores provided to the pressing member 52, and the pressing member 52 is disposed to be movable in a perpendicular direction along the guide pins 55. It is also preferred that an opening portion is provided to the top panel 54 such that the inside of the cell culture jig 5 is visible through the opening portion.

The stage 51 is formed of a substrate enabling ventilation between the gas permeable film and the stage 51, for example, preferably formed by uneven processing of a surface of the substrate, and more preferably includes a protruding portion 511 formed by the uneven processing on the substrate surface.

The pressing member 52 is formed of a substrate enabling ventilation between the gas permeable film and the pressing member 52, for example, preferably formed by uneven processing of the substrate surface, and more preferably includes a protruding portion 521 formed by the uneven processing on the substrate surface.

As the protruding portion 511 and the protruding portion 521, as illustrated in FIG. 6(b), FIG. 7, and FIG. 8, those formed by arranging a plurality of approximately triangular prisms in parallel in a mountain range shape (hereinafter referred to as a mountain range pattern in some cases) are preferable.

By thus forming the protruding portion 511 and the protruding portion 521 in the mountain range patterns, contacted areas of the gas permeable film with the stage 51 and the pressing member 52 can be reduced, and the inhibition of the gas permeation performance of the cell culture vessel 6 can be avoided.

With the mountain range pattern, strengths of the protrusions of the protruding portion 511 and the protruding portion 521 can be enhanced, a damage such as a distortion of the protrusion due to the pressing force of the pressing member 52 can be avoided.

Furthermore, flat portions are disposed between the plurality of approximately triangular prisms in the mountain range pattern, thereby allowing ensuring a visibility inside the cell culture vessel 6 when the gas permeable film is a transparent material.

As the above-described substrate enabling ventilation, using a porous material is preferable. Furthermore, as the above-described substrate enabling ventilation, using a perforated plate is also preferable.

In the cell culture jig 5 of the embodiment, the inhibition of the gas permeation performance of the cell culture vessel can be suppressed even when a porous material or a perforated plate is used as the substrate enabling ventilation.

It is also preferred that the cell culture jig 5 of the embodiment includes permanent magnets (not illustrated) as biasing means that bias the pressing member 52 downward at four corners of the top panel 54, and includes permanent magnets as biasing means at positions of the pressing member 52 corresponding to the permanent magnets while the same pole sides are mutually opposed.

By using the biasing means, the pressing member 52 is configured to be movable in the perpendicular direction by a repulsive force acting between the permanent magnets in a state of perpendicularly pressing planar substrates 61 of the cell culture vessel 6.

The biasing means is not limited to the permanent magnet, and the pressing member 52 may be configured to be moved up and down by the own weight of the pressing member 52 without the biasing means.

The stage 51 can be formed of, for example, a synthetic resin such as polycarbonate.

The pressing member 52 may be formed of, for example, a synthetic resin such as polycarbonate, or may be formed of a glass. As the pressing member 52, for confirming the progress status of the culture, the state of the culture object, and the like, using one partially or entirely having transparency is preferable.

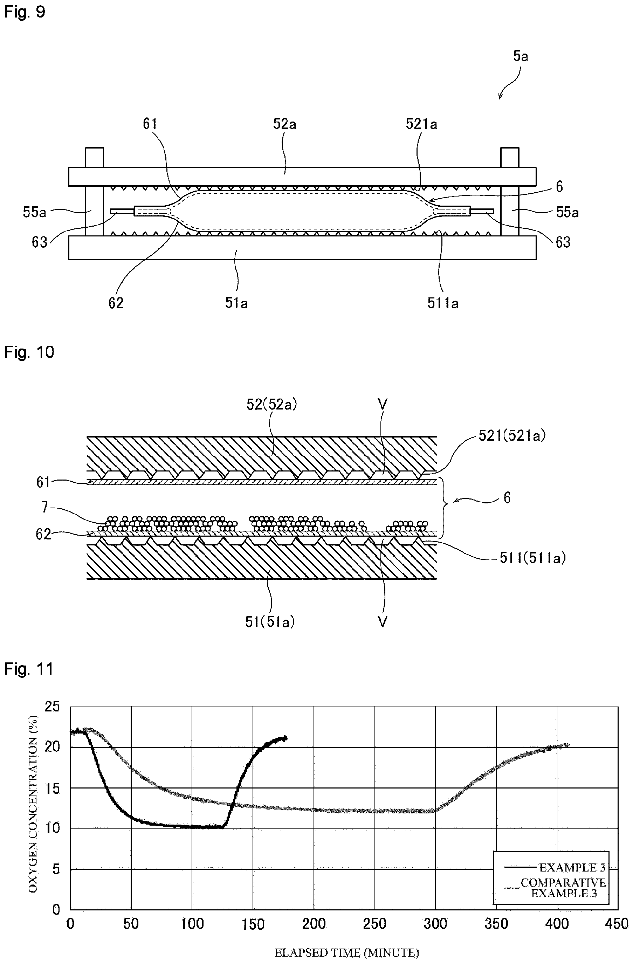

Next, a modification of the cell culture jig of the embodiment will be described with reference to FIG. 9.

As illustrated in the drawing, the cell culture vessel 6 is placed on a stage 51a of a cell culture jig 5a, and the upper surface of the cell culture vessel 6 is pressed by a pressing member 52a.

Guide pins 55a are disposed upright on four corners of the stage 51a, and the guide pins 55a pass through guide bores provided to the pressing member 52a. Then, the pressing member 52a is disposed to be movable in the perpendicular direction along the guide pins 55a.

At this time, the pressing member 52a can be fixed by the own weight so as to press the cell culture vessel 6 with a constant pressure. Spring materials may be installed to the guide pins 55a to appropriately press the pressing member 52a to the cell culture vessel 6. Accordingly, the pressing member 52a can be configured to be movable in the perpendicular direction in accordance with injection/discharge of the culture fluid to/from the cell culture vessel 6.

In the embodiment, while the cell culture vessel 6 is not especially limited, the cell culture vessel 6 can be obtained by, for example, thermally welding peripheral edge portions of two rectangular planar substrates 61, 62 by a heat seal. A space formed between the two planar substrates is used as a culture space for culturing cells. The regions forming the culture space of the planar substrates 61, 62 constitute a culture portion in the cell culture vessel 6.

At least one of the planar substrates 61, 62 is formed of a gas permeable film, and it is preferred to use one in which both are formed of the gas permeable film. As the gas permeable film, a polyolefin-based resin, for example, polyethylene, such as linear low density polyethylene (LLDPE), or polypropylene can be preferably used. To make the inside of the cell culture vessel 6 visible, the gas permeable film is preferably a transparent material.

The "planar" of the planar substrates 61, 62 does not mean that the substrate surface is flat without unevenness, but means that the entire shape of the substrate is roughly flat, and includes one in which the unevenness is formed on the substrate surface.

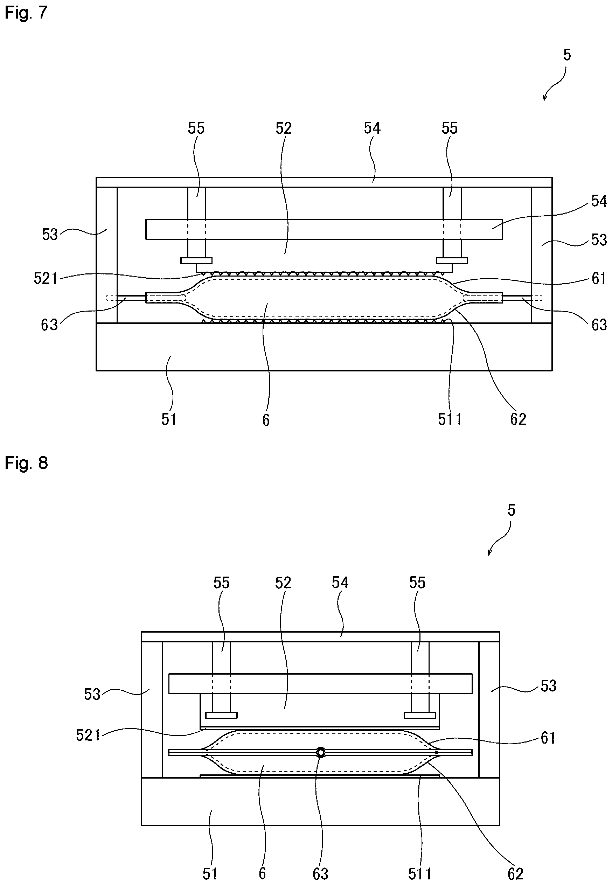

When the cell culture vessel 6 is filled with the culture fluid, as illustrated in FIG. 7 and FIG. 8, the pressing member 52 is pushed up by the cell culture vessel 6.

On the other hand, when the culture fluid is discharged from the cell culture vessel 6 through ports 63, a thickness of the culture fluid in the cell culture vessel 6 decreases in accordance with the discharge, and accordingly, the pressing member 52 moves down.

While the two ports 63 are disposed to be mutually opposed at both ends in a longitudinal direction of the cell culture vessel 6 in FIG. 7, the number of the ports is not limited thereto, and the number of the ports may be one, or three or more. As the material of the port, for example, a thermoplastic resin, such as polyethylene, polypropylene, vinyl chloride, polystyrene-based elastomer, or FEP can be used.

FIG. 10 illustrates a state of the cell culture using the cell culture jig 5 (5a) of the embodiment.

The drawing illustrates a state of performing the culture in which a culture fluid is filled in the cell culture vessel 6, and cells are seeded in the culture space whose volume has been enlarged by the culture fluid.

A ventilation space V is formed between the protruding portion 511 (511a) on the stage 51 (51a) of the cell culture jig 5 (5a) and a planar substrate 62 in the stage side of the cell culture vessel 6.

A ventilation space V is formed between the protruding portion 521 (521a) on the pressing member 52 (52a) of the cell culture jig 5 (5a) and a planar substrate 61 in the top panel side of the cell culture vessel 6 as well.

Thus, with the cell culture jig 5 (5a) of the embodiment, the inhibition of the gas permeation performance of the cell culture vessel 6 can be suppressed in performing the cell culture, and cells can be cultured with a high density.

In a method for producing cells of the embodiment, cells are cultured using the above-described cell culture jig 5.

The cells cultured using the cell culture jig 5 are not especially limited, and the cells may be floating cells, such as lymphocytes or dendritic cells, cultured by floating cells in a culture fluid, or may be adherent cells, such as induced pluripotent stem cells (iPS cells), neural stem cells, embryonic stem cells (ES cells), mesenchymal stem cells, hepatic cells, islet cells, cardiac muscle cells, corneal endothelial cells, and lymph corpuscles in an activation process, cultured by making cells adhere to a culture portion in the vessel.

As described above, with the cell culture jig of the embodiment, the inhibition of the gas permeation performance of the cell culture vessel can be suppressed, and cells can be cultured with a high density.

Next, embodiments of a cell culture vessel, a method for producing cells, and a method for producing a cell culture vessel of the present invention will be described in detail. However, the present invention is not limited to the specific contents of the embodiments below.

### [Third Embodiment]

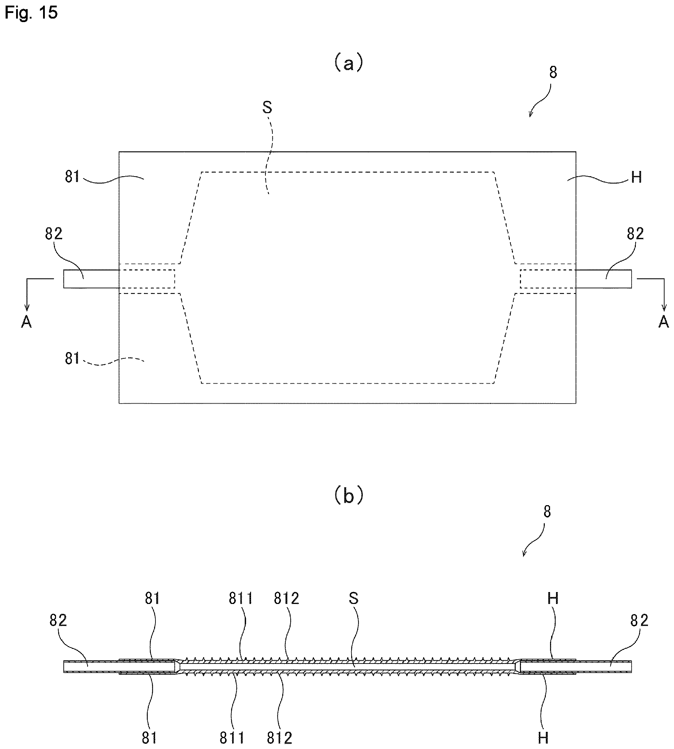

First, the third embodiment of the present invention will be described with reference to FIG. 15 to FIG. 18.

A cell culture vessel of the embodiment is a bag-shaped cell culture vessel of a closed system, and the cell culture vessel includes at least one port and mutually opposed planar substrates. At least one of the planar substrates is formed of a gas permeable film. A culture space for culturing cells is provided in a vessel inner surface side of the gas permeable film. A plurality of thin portions and projecting portions are formed on the gas permeable film forming the culture space. The thin portion has a thickness of 75 µm or less. It is also preferred that the thin portion has the thickness of 20 µm to 35 µm.

Specifically, as illustrated in FIG. 15(a), a cell culture vessel 8 of the embodiment can be obtained by, for example, thermally welding peripheral edge portions H of two rectangular planar substrates 81 by a heat seal. A space formed between the two planar substrates 81 is used as a culture space S for culturing cells. The regions forming the culture space S of the planar substrates 81 constitute a culture portion in the cell culture vessel 8. The entire shape of the planar substrate 81, the shape of the peripheral edge portion H, and the shape of the culture space S are not especially limited, and those having any shapes can be used.

At least one of the planar substrates 81 is formed of a gas permeable film, and it is preferred to use one in which both are formed of the gas permeable film.

The "planar" of the planar substrate 81 does not mean that the substrate surface is flat without unevenness, but means that the entire shape of the substrate is roughly flat, and includes one in which the unevenness is formed on the surface of the planar substrate 81.

As illustrated in FIG. 15(b), in the cell culture vessel 8 of the embodiment, a plurality of thin portions 811 are formed on the planar substrate 81 as the gas permeable film. A plurality of projecting portions 812 are formed on a vessel outer surface of the gas permeable film. Furthermore, the vessel inner surface of the gas permeable film is flat without unevenness.

The number of the projecting portions formed on the gas permeable film is not limited to a plural number, and one in which a plurality of projecting portions are mutually connected to form substantially one projecting portion like a projecting portion in a cell culture vessel of a fourth embodiment or the like is included.

While both of the mutually opposed planar substrates 81 are formed of the gas permeable film, and the thin portions 811 and the projecting portions 812 are formed on each of the gas permeable films in this embodiment, a configuration in which only any one of the planar substrates 81 is formed of the gas permeable film, and the thin portions 811 and the projecting portions 812 are formed on the gas permeable film may be employed.

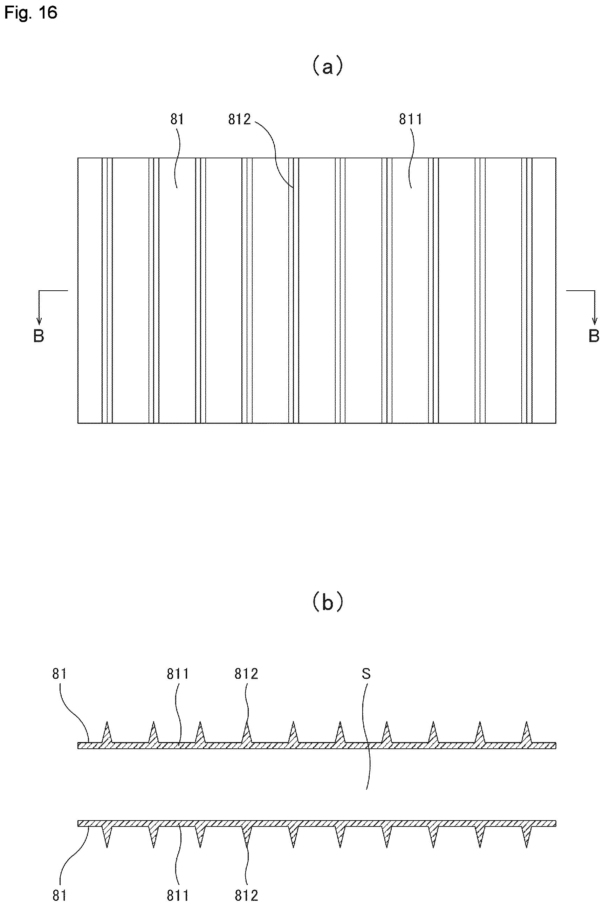

As illustrated in FIG. 16(a) and FIG. 16(b), the projecting portion 812 preferably has a shape formed by arranging a plurality of approximately triangular prisms in parallel in a mountain range shape (hereinafter referred to as a mountain range pattern in some cases).

By thus forming the projecting portions 812 in the mountain range pattern, a contacted area of the gas permeable film with a planar surface can be reduced, and the inhibition of the gas permeation performance of the cell culture vessel 8 can be avoided.

With the mountain range pattern, strengths of the protrusions of the projecting portions 812 can be enhanced.

In the cell culture vessel 8 of the embodiment, the projecting portions 812 mutually adjacent in parallel are formed with intervals of 1 mm to 5 mm. That is, the projecting portions 812 are formed so as to have a width (width in shorter direction) of the thin portion 811 of 1 mm to 5 mm. Accordingly, the strength in handling and the gas permeation performance of the cell culture vessel 8 can be made sufficient. For making the strength in handling and the gas permeation performance of the cell culture vessel 8 more appropriate, the width of the thin portion 811 is preferably 1 mm to 3 mm, and more preferably 1 mm to 2 mm.

The interval between the projecting portions 812 mutually adjacent in parallel may be smaller than 1 mm, and as described in a fifth embodiment or the like below, even when the projecting portions 812 mutually adjacent in parallel are formed without intervals, not only the strength in handling can be ensured, but also a magnitude of gas permeability capable of culturing cells with a high density can be provided.

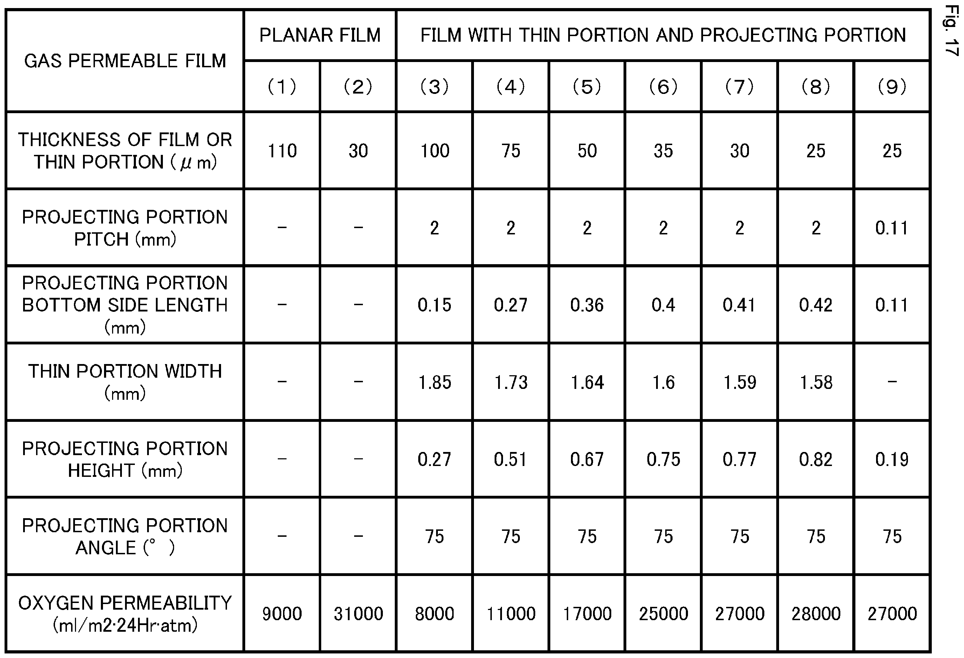

Here, with reference to FIG. 17, oxygen permeabilities of the gas permeable film on which a plurality of thin portions and projecting portions are formed and the like will be described.

First, as the gas permeable films, two types having a planar shape without unevenness and seven types with thin portions and projecting portions were prepared.

Specifically, a planar film (1) having a film thickness of 110 µm and a planar film (2) having a film thickness of 30 µm, which were formed of a linear low density polyethylene (LLDPE), were prepared.

Additionally, LLDPE films (3) to (8) with thin portions and projecting portions were prepared. The thicknesses of the thin portions were 100 µm, 75 µm, 50 µm, 35 µm, 30 µm, and 25 µm, respectively. In each of the films (3) to (8), the pitch of the projecting portion was 2 mm. The bottom side lengths of the projecting portions were 0.15 mm, 0.27 mm, 0.36 mm, 0.4 mm, 0.41 mm, and 0.42 mm, respectively. The thin portion widths were 1.85 mm, 1.73 mm, 1.64 mm, 1.6 mm, 1.59 mm, and 1.58 mm, respectively. The projecting portion heights were 0.27 mm, 0.51 mm, 0.67 mm, 0.75 mm, 0.77 mm, and 0.82 mm, respectively. The projecting portion angle (angle between bottom side and oblique side of projecting portion) was 75° in each of the films (3) to (8).

Furthermore, a LLDPE film (9) with thin portions and projecting portions was prepared. In the film (9), the projecting portions mutually adjacent in parallel were continuously formed without intervals (thin portion thickness was 25 µm, pitch between projecting portions was 0.11 mm, length of projecting portion bottom side was 0.11 mm, projecting portion height was 0.19 mm, and projecting portion angle was 75°).

The films (3) to (9) were each formed by processing each projecting portion onto a planar LLDPE film having the film thickness of 110 µm by thermal transfer.

Then, the oxygen permeability was measured based on the flow method (equal-pressure method, JIS K 7126-2) using a gas permeability measurement device (manufactured by GTR TEC Corporation, Flow method Gas and Water Vapor Permeation Analysis System GTR-20X).

As illustrated in FIG. 17, the oxygen permeabilities (ml/m²·24Hr·atm) of the planar films having the film thicknesses of 110 µm and 30 µm were 9000 and 31000, respectively, while the oxygen permeabilities of the films with thin portions and projecting portions (thin portion thickness: 100 µm, 75 µm, 50 µm, 35 µm, 30 µm, 25 µm, and 25 µm (continuous projecting portion)) were 8000, 11000, 17000, 25000, 27000, 28000, and 27000, respectively.

Thus, by including the projecting portions, the film with thin portions and projecting portions can not only ensure the strength in handling, but also provide the oxygen permeability equal to or more than that of the planar film having the thickness of 110 µm. That is, while these films are obtained by processing the planar film having the thickness of 110 µm as described above, forming the thin portions and the projecting portions enables the oxygen permeability to be more improved.

Especially, those having the thin portion thickness of 75 µm or less can provide the oxygen permeability of 11000 or more, and those having the thin portion thickness of 35 µm to 25 µm can provide the excellent oxygen permeability of 25000 or more.

The oxygen permeability is similar in the case where the projecting portions are formed in the vessel outer side of the gas permeable film and in the case where the projecting portions are formed in the vessel inner side of the gas permeable film.

As the planar substrate 81, a resin film or the like can be preferably used, and for example, a polyolefin-based resin such as polyethylene and polypropylene is usable. For example, polyethylene, a copolymer of ethylene and α-olefin, a copolymer of ethylene and vinyl acetate, an ionomer using: a copolymer of ethylene and an acrylic acid or ethylene and a methacrylic acid; and a metal ion, and the like can be listed. Further, a polyolefin, a styrene-based elastomer, a polyester-based thermoplastic elastomer, and the like can be used. Furthermore, a soft vinyl chloride resin, a polybutadiene resin, an ethylene-vinyl acetate copolymer, a chlorinated polyethylene resin, a polyurethane-based thermoplastic elastomer, a polyester-based thermoplastic elastomer, a silicone-based thermoplastic elastomer, a styrene-based elastomer, such as styrene-butadiene-styrene (SBS), styrene-isoprene-styrene (SIS), styrene-ethylene-butylene-styrene (SEBS), and styrene-ethylene-propylene-styrene (SEPS), a polyolefin resin, a fluorine-based resin, and the like may be used.

As the gas permeable film, among the above-described materials of the planar substrate 81, especially, using a thermoplastic resin excellent in gas permeation performance is preferable, and a polyolefin-based resin, for example, polyethylene, such as LLDPE, or polypropylene can be preferably used. To make the inside of the cell culture vessel 8 visible, the gas permeable film is preferably a transparent material.

Here, for the gas permeable film, generally, the smaller the thickness is, the higher the gas permeation performance becomes, while the smaller the thickness of the gas permeable film is, the more difficult its handling becomes. Especially, since one having the thickness of 30 µm or 20 µm is like a food wrap (thickness about 10 µm), the vessel strength is insufficient, thus possibly causing a break.

However, since the cell culture vessel 8 of the embodiment includes the projecting portions 812 on the gas permeable film, the cell culture vessel 8 can be handled even when the thickness of the thin portion 811 of the gas permeable film is relatively thin.

In view of this, the thickness of the gas permeable film in the cell culture vessel 8 of the embodiment is preferably less than 100 µm, more preferably 75 µm or less, and further preferably 35 µm or less and 20 µm or more.

The cell culture vessel 8 of the embodiment includes at least one port 82 sandwiched between the peripheral edge portions H of the two planar substrates 81 and thermally welded.

While the two ports 82 are disposed to be mutually opposed at both ends in a longitudinal direction of the planar substrate 81 in FIG. 15, the number of the ports 82 is not limited thereto, and the number of the ports 82 may be one, or three or more.

As the material of the port 82, for example, a thermoplastic resin, such as polyethylene, polypropylene, vinyl chloride, polystyrene-based elastomer, or FEP can be used.

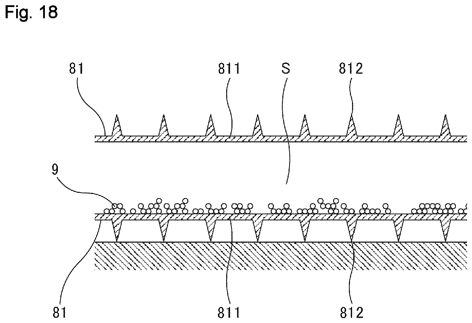

FIG. 18 illustrates a state of a cell culture by the cell culture vessel 8 of the embodiment.

The drawing illustrates a state of performing the culture in which a culture fluid is filled in the cell culture vessel 8, and cells 9 are seeded in the culture space S whose volume has been enlarged by the culture fluid.

The cells cultured using the cell culture vessel 8 are not especially limited, and the cells may be floating cells, such as lymphocytes or dendritic cells, cultured by floating cells in a culture fluid, or may be adherent cells, such as induced pluripotent stem cells (iPS cells), neural stem cells, embryonic stem cells (ES cells), mesenchymal stem cells, hepatic cells, islet cells, cardiac muscle cells, corneal endothelial cells, and lymph corpuscles in an activation process, cultured by making cells adhere to a culture portion in the vessel.

In a method for producing cells of the embodiment, cells are cultured using the above-described cell culture vessel 8. Also in methods for producing cells in embodiments described later, similarly, while cells are cultured using cell culture vessels of the respective embodiments, their descriptions are omitted below.

A method for producing a cell culture vessel of the embodiment is a method for producing a bag-shaped cell culture vessel 8 of a closed system, and the cell culture vessel 8 includes at least one port 82 and mutually opposed planar substrates 81. A gas permeable film is used for at least one of the planar substrates 81. A plurality of thin portions 811 having thicknesses of 75 µm or less and projecting portions 812 are formed on the gas permeable film. A culture space S for culturing cells 9 is formed in the vessel inner surface side of the gas permeable film by bonding peripheral edge portions H of the mutually opposed planar substrates 81.

In the method for producing a cell culture vessel of the embodiment, it is also preferred that a plurality of thin portions 811 having thicknesses of 20 µm to 35 µm and projecting portions 812 are formed on the gas permeable film.

Furthermore, in the method for producing a cell culture vessel of the embodiment, it is also preferred that the projecting portions 812 are formed on a vessel outer surface of the gas permeable film, and a vessel inner surface of the gas permeable film is flat without unevenness.

In the method for producing a cell culture vessel of the embodiment, it is also preferred that the gas permeable film is used for both of the mutually opposed planar substrates 81, and a plurality of thin portions 811 and projecting portions 812 are formed on each of the gas permeable films.

Furthermore, in the method for producing a cell culture vessel of the embodiment, it is also preferred that the projecting portions 812 are formed by arranging a plurality of approximately triangular prisms in parallel in a mountain range shape.

In the method for producing a cell culture vessel of the embodiment, it is also preferred that the projecting portions 812 mutually adjacent in parallel are formed with intervals of 1 mm to 5 mm.

Furthermore, in the method for producing a cell culture vessel of the embodiment, it is also preferred that the projecting portions 812 are formed by a thermal transfer or a cast film forming.

Thus, according to the cell culture vessel, the method for producing cells, and the method for producing a cell culture vessel of the embodiment, the cell culture vessel in which the projecting portions are formed with intervals in a mountain range pattern on the outer side of the vessel can be produced, and the cell culture vessel is excellent in gas permeation performance and strength. Using the cell culture vessel enables efficiently culturing cells in a large amount.

### [Fourth Embodiment]

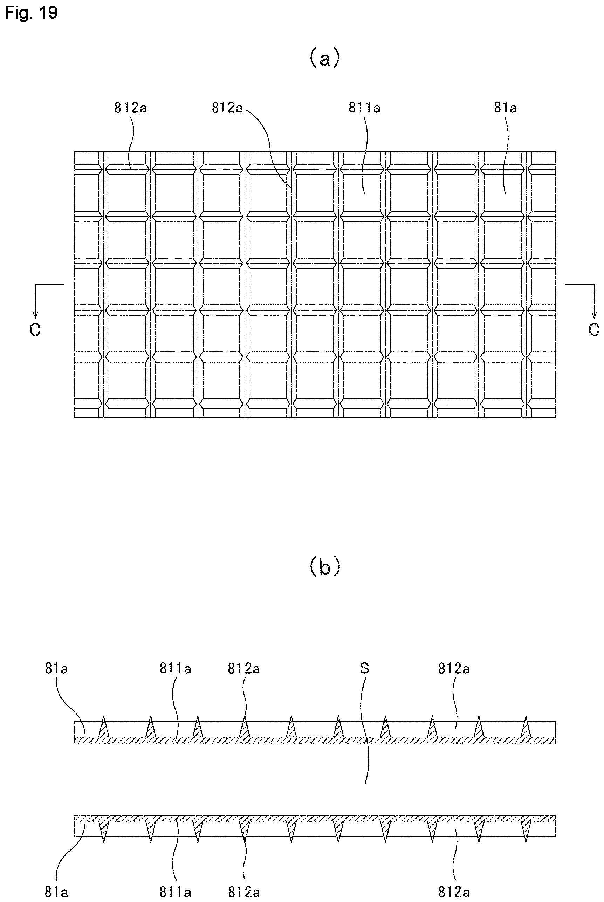

Next, the fourth embodiment of the present invention will be described with reference to FIG. 19.

A cell culture vessel of the embodiment is different from the third embodiment in that a plurality of thin portions 811a and projecting portions 812a are formed on the gas permeable film, and as the projecting portions 812a, a plurality of approximately triangular prisms are formed to be arranged in parallel in a mountain range pattern on a vessel outer surface of the gas permeable film, a plurality of other approximately triangular prisms are formed to be arranged in parallel in a mountain range pattern so as to intersect with the plurality of approximately triangular prisms, thus forming these plurality of approximately triangular prisms in a grid pattern.

Other configurations of the cell culture vessel, the method for producing cells, and the method for producing a cell culture vessel of the embodiment are similar to those in the third embodiment excluding the points described below.

Specifically, as illustrated in FIG. 19(a) and FIG. 19(b), in the cell culture vessel of the embodiment, a plurality of thin portions 811a are formed on a planar substrate 81a as the gas permeable film. Projecting portions 812a are formed on a vessel outer surface of the gas permeable film. Furthermore, a vessel inner surface of the gas permeable film is flat without unevenness.

While both of the mutually opposed planar substrates 81a are formed of the gas permeable film, and the thin portions 811a and the projecting portions 812a are formed on each of the gas permeable films in this embodiment, a configuration in which only any one of the planar substrates 81a is formed of the gas permeable film, and the thin portions 811a and the projecting portions 812a are formed on the gas permeable film may be employed.

The projecting portions 812a are preferably formed by arranging a plurality of approximately triangular prisms in parallel in a mountain range pattern and arranging a plurality of other approximately triangular prisms in parallel in a mountain range pattern so as to intersect with the plurality of approximately triangular prisms (hereinafter referred to as a grid pattern in some cases).

In FIG. 19(a) and FIG. 19(b), the heights of the approximately triangular prisms in the horizontal direction are formed to be lower than the heights of the approximately triangular prisms in the vertical direction.

Accordingly, when the cell culture vessel of the embodiment is placed on a planar surface while having the surface on which the projecting portions 812a are formed facing downward, a space enabling ventilation is formed between the planar substrate 81a and the planar surface.

Similarly, when the cell culture vessel of the embodiment is pressed from the upper side using a pressing plate or the like while having the surface on which the projecting portions 812a are formed facing upward, a space enabling ventilation is formed between the planar substrate 81a and the planar surface.

Thus, a space enabling ventilation is formed between the cell culture vessel and the planar surface by forming the projecting portions on the cell culture vessel, and the same applies to the embodiments below.

By thus forming the projecting portions 812a in a grid pattern, the contacted area of the gas permeable film with the planar surface can be reduced, and the inhibition of the gas permeation performance of the cell culture vessel can be avoided.

With the grid pattern, strengths of the protrusions of the projecting portions 812a can be enhanced.

Further, in FIG. 19(a), while the plurality of approximately triangular prisms are perpendicularly intersected with the plurality of other approximately triangular prisms as the projecting portions 812a, the projecting portions 812a may be formed by obliquely intersecting the approximately triangular prisms.

In the cell culture vessel of the embodiment, the projecting portions 812a mutually adjacent in parallel are formed with intervals of 1 mm to 5 mm. That is, while the thin portion 811a are formed so as to have a width of 1 mm to 5 mm, the width of the thin portion 811a is preferably 1 mm to 3 mm, and more preferably 1 mm to 2 mm.

In the method for producing a cell culture vessel of the embodiment, it is preferred that as the projecting portions 812a, a plurality of approximately triangular prisms are formed to be arranged in parallel in a mountain range pattern, a plurality of other approximately triangular prisms are formed to be arranged in parallel in a mountain range pattern so as to intersect with the plurality of approximately triangular prisms, and these plurality of approximately triangular prisms are formed in a grid pattern.

Thus, according to the cell culture vessel, the method for producing cells, and the method for producing a cell culture vessel of the embodiment, the cell culture vessel in which the projecting portions are formed with intervals in a grid pattern on the outer side of the vessel can be produced, and the cell culture vessel is excellent in gas permeation performance and strength. Using the cell culture vessel enables efficiently culturing cells in a large amount.

### [Fifth Embodiment]

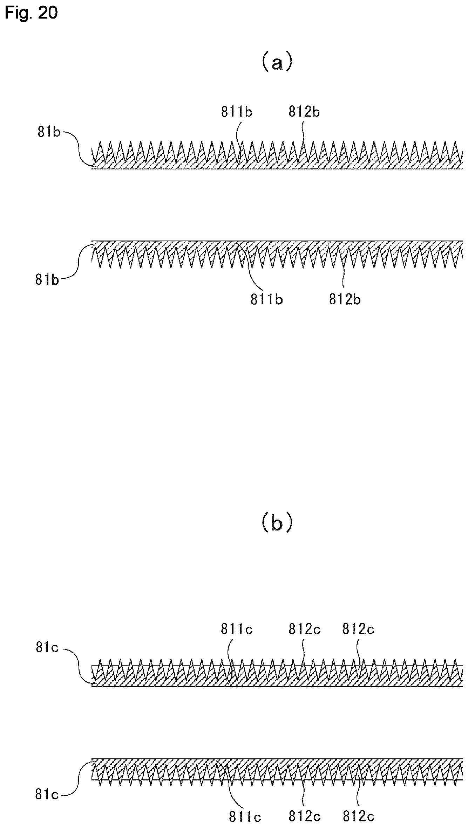

Next, the fifth embodiment of the present invention will be described with reference to FIG. 20(a).

A cell culture vessel of the embodiment is different from the third embodiment in that a plurality of thin portions 811b and projecting portions 812b are formed on the gas permeable film, and as the projecting portions 812b, a plurality of approximately triangular prisms are formed to be arranged in parallel in a mountain range pattern on a vessel outer surface of the gas permeable film, and the projecting portions 812b mutually adjacent in parallel are formed without intervals, thus forming the thin portions 811b in line shapes.

Other configurations of the cell culture vessel, the method for producing cells, and the method for producing a cell culture vessel of the embodiment are similar to those in the third embodiment excluding the points described below.

Specifically, as illustrated in FIG. 20(a), in the cell culture vessel of the embodiment, a plurality of thin portions 811b are formed to be parallel in line shapes (in the drawing, in depth direction) on a planar substrate 81b as the gas permeable film. Projecting portions 812b are continuously formed to be parallel without intervals in a front-back direction (in the drawing, depth direction) on a vessel outer surface of the gas permeable film. Furthermore, a vessel inner surface of the gas permeable film is flat without unevenness.

While both of the mutually opposed planar substrates 81b are formed of the gas permeable film, and the thin portions 811b and the projecting portions 812b are formed on each of the gas permeable films in this embodiment, a configuration in which only any one of the planar substrates 81b is formed of the gas permeable film, and the thin portions 811b and the projecting portions 812b are formed on the gas permeable film may be employed.

As illustrated in FIG. 20(a), the projecting portions 812b are preferably formed by arranging a plurality of approximately triangular prisms in parallel in a mountain range pattern (mountain range pattern).

By thus forming the projecting portions 812b in the mountain range pattern, a contacted area of the gas permeable film with a planar surface can be reduced, and the inhibition of the gas permeation performance of the cell culture vessel can be avoided.

With the mountain range pattern, strengths of the protrusions of the projecting portions 812b can be enhanced.

In the method for producing a cell culture vessel of the embodiment, it is preferred that as the projecting portions 812b, projecting portions mutually adjacent in parallel are formed without intervals, thus forming the thin portions 811b in line shapes.

Thus, according to the cell culture vessel, the method for producing cells, and the method for producing a cell culture vessel of the embodiment, the cell culture vessel in which the projecting portions are formed without intervals in a mountain range pattern on the outer side of the vessel can be produced, and the cell culture vessel is excellent in gas permeation performance and strength. Using the cell culture vessel enables efficiently culturing cells in a large amount.

### [Sixth Embodiment]

Next, the sixth embodiment of the present invention will be described with reference to FIG. 20(b).

A cell culture vessel of the embodiment is different from the third embodiment in that a plurality of thin portions 811c and projecting portions 812c are formed on the gas permeable film, and as the projecting portions 812c, a plurality of approximately triangular prisms are disposed in a grid pattern on a vessel outer surface of the gas permeable film, and the projecting portions 812c mutually adjacent in parallel are formed without intervals, thus forming the thin portions 811c in dot shapes.

Other configurations of the cell culture vessel, the method for producing cells, and the method for producing a cell culture vessel of the embodiment are similar to those in the third embodiment excluding the points described below.

Specifically, as illustrated in FIG. 20(b), in the cell culture vessel of the embodiment, a plurality of thin portions 811c are formed in dot shapes on a planar substrate 81c as the gas permeable film. Projecting portions 812c are continuously formed to be parallel without intervals in a front-back direction (in the drawing, depth direction) and a right-left direction on a vessel outer surface of the gas permeable film. Furthermore, a vessel inner surface of the gas permeable film is flat without unevenness.

While both of the mutually opposed planar substrates 81c are formed of the gas permeable film, and the thin portions 811c and the projecting portions 812c are formed on each of the gas permeable films in this embodiment, a configuration in which only any one of the planar substrates 81c is formed of the gas permeable film, and the thin portions 811c and the projecting portions 812c are formed on the gas permeable film may be employed.

The projecting portions 812c are preferably formed by arranging a plurality of approximately triangular prisms in parallel in a mountain range pattern and arranging a plurality of other approximately triangular prisms in parallel in a mountain range pattern so as to intersect with the plurality of approximately triangular prisms (grid pattern).

In FIG. 20(b), the heights of the approximately triangular prisms in the horizontal direction are formed to be lower than the heights of the approximately triangular prisms in the vertical direction (in the drawing, depth direction). Accordingly, when the cell culture vessel of the embodiment is placed on a planar surface, a space enabling ventilation is formed between the planar substrate 81c and the planar surface.

By thus forming the projecting portions 812c in a grid pattern, the contacted area of the gas permeable film with the planar surface can be reduced, and the inhibition of the gas permeation performance of the cell culture vessel can be avoided.

With the grid pattern, strengths of the protrusions of the projecting portions 812c can be enhanced.

Further, the projecting portions 812c may be formed such that the plurality of approximately triangular prisms obliquely intersect with the plurality of other approximately triangular prisms.

In the method for producing a cell culture vessel of the embodiment, it is preferred that as the projecting portions 812c, a plurality of approximately triangular prisms are formed to be arranged in parallel in a mountain range pattern, a plurality of other approximately triangular prisms are formed to be arranged in parallel in a mountain range pattern so as to intersect with the plurality of approximately triangular prisms, and these plurality of approximately triangular prisms are formed in a grid pattern. Additionally, it is preferred that the projecting portions 812c mutually adjacent in parallel are formed without intervals, thus forming the thin portions 811c in dot shapes.

Thus, according to the cell culture vessel, the method for producing cells, and the method for producing a cell culture vessel of the embodiment, the cell culture vessel in which the projecting portions are formed without intervals in a grid pattern on the outer side of the vessel can be produced, and the cell culture vessel is excellent in gas permeation performance and strength. Using the cell culture vessel enables efficiently culturing cells in a large amount.

### [Seventh Embodiment]

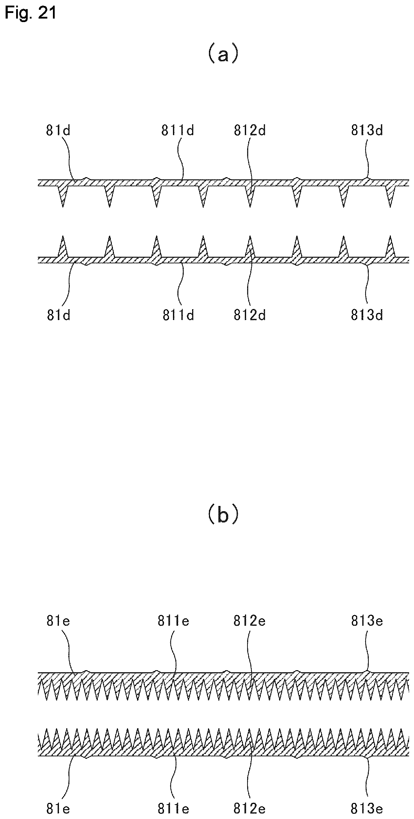

Next, the seventh embodiment of the present invention will be described with reference to FIG. 21(a).

A cell culture vessel of the embodiment is different from the third embodiment in that: a plurality of thin portions 811d and projecting portions 812d are formed on the gas permeable film; as the projecting portions 812d, a plurality of approximately triangular prisms are formed to be arranged in parallel in a mountain range pattern on a vessel inner surface of the gas permeable film, and the projecting portions 812d mutually adjacent in parallel are formed with intervals; a plurality of small protruding portions 813d are formed on a vessel outer surface of the gas permeable film; and a space enabling ventilation is formed between the gas permeable film and a planar surface by the small protruding portions 813d when the outer surface is brought in contact with the planar surface.

Other configurations of the cell culture vessel, the method for producing cells, and the method for producing a cell culture vessel of the embodiment are similar to those in the third embodiment excluding the points described below.

Specifically, as illustrated in FIG. 21(a), in the cell culture vessel of the embodiment, a plurality of thin portions 811d are formed on a planar substrate 81d as the gas permeable film. Projecting portions 812d are formed in parallel with each other with intervals in a front-back direction (in the drawing, depth direction) on a vessel inner surface of the gas permeable film. Furthermore, a plurality of small protruding portions 813d are formed on a vessel outer surface of the gas permeable film.

The shape of the small protruding portion 813d is not especially limited insofar as a space enabling ventilation can be formed between the gas permeable film and the planar surface, and various shapes including a conical shape, a triangular pyramid shape, a polygonal pyramid shape, a rectangular parallelepiped shape, a mountain range pattern, a grid pattern, and the like may be employed. The same applies to small protruding portions of embodiments below.

While both of the mutually opposed planar substrates 81d are formed of the gas permeable film, and the thin portions 811d, the projecting portions 812d, and the small protruding portions 813d are formed on each of the gas permeable films in this embodiment, a configuration in which only any one of the planar substrates 81d is formed of the gas permeable film, and the thin portions 811d, the projecting portions 812d, and the small protruding portions 813d are formed on the gas permeable film may be employed.

As illustrated in FIG. 21(a), the projecting portions 812d are preferably formed by arranging a plurality of approximately triangular prisms in parallel in a mountain range pattern (mountain range pattern).

By thus forming the projecting portions 812d in the mountain range pattern, a contacted area of the gas permeable film with a planar surface can be reduced, and the inhibition of the gas permeation performance of the cell culture vessel can be avoided.

With the mountain range pattern, strengths of the protrusions of the projecting portions 812d can be enhanced.

While the thin portion 811d is formed so as to have a width of 1 mm to 5 mm in the embodiment, the width of the thin portion 811d is preferably 1 mm to 3 mm, and more preferably 1 mm to 2 mm.

In the method for producing a cell culture vessel of the embodiment, it is preferred that as the projecting portions 812d, a plurality of approximately triangular prisms are formed to be arranged in parallel with each other with intervals in a mountain range pattern on a vessel inner surface of the gas permeable film, a plurality of small protruding portions 813d are formed on a vessel outer surface of the gas permeable film, and when the outer surface is brought in contact with a planar surface, a space enabling ventilation is formed between the gas permeable film and the planar surface by the small protruding portions 813d.

Thus, according to the cell culture vessel, the method for producing cells, and the method for producing a cell culture vessel of the embodiment, the cell culture vessel in which the projecting portions are formed with intervals in a mountain range pattern on the inner side of the vessel can be produced, and the cell culture vessel is excellent in gas permeation performance and strength. Using the cell culture vessel enables efficiently culturing cells in a large amount.

### [Eighth Embodiment]

Next, the eighth embodiment of the present invention will be described with reference to FIG. 21(b).

A cell culture vessel of the embodiment is different from the seventh embodiment in that a plurality of thin portions 811e and projecting portions 812e are formed on the gas permeable film, and as the projecting portions 812e, a plurality of approximately triangular prisms are formed to be arranged in parallel in a mountain range pattern on a vessel inner surface of the gas permeable film, and the projecting portions 812e mutually adjacent in parallel are formed without intervals.

Other configurations of the cell culture vessel, the method for producing cells, and the method for producing a cell culture vessel of the embodiment are similar to those in the seventh embodiment excluding the points described below.

Specifically, as illustrated in FIG. 21(b), in the cell culture vessel of the embodiment, a plurality of thin portions 811 e are formed to be parallel in line shapes (in the drawing, in depth direction) on a planar substrate 81e as the gas permeable film. Projecting portions 812e are formed to be parallel without intervals in a front-back direction (in the drawing, depth direction) on a vessel inner surface of the gas permeable film. Furthermore, a plurality of small protruding portions 813e are formed on a vessel outer surface of the gas permeable film.

While both of the mutually opposed planar substrates 81e are formed of the gas permeable film, and the thin portions 811e, the projecting portions 812e, and the small protruding portions 813e are formed on each of the gas permeable films in this embodiment, a configuration in which only any one of the planar substrates 81e is formed of the gas permeable film, and the thin portions 811e, the projecting portions 812e, and the small protruding portions 813e are formed on the gas permeable film may be employed.

As illustrated in FIG. 21(b), the projecting portions 812e are preferably formed by arranging a plurality of approximately triangular prisms in parallel in a mountain range pattern (mountain range pattern).

By thus forming the projecting portions 812e in the mountain range pattern, a contacted area of the gas permeable film with a planar surface can be reduced, and the inhibition of the gas permeation performance of the cell culture vessel can be avoided.

With the mountain range pattern, strengths of the protrusions of the projecting portions 812e can be enhanced.

In the method for producing a cell culture vessel of the embodiment, it is preferred that as the projecting portions 812e, a plurality of approximately triangular prisms are formed to be arranged in parallel without intervals in a mountain range pattern on a vessel inner surface of the gas permeable film, a plurality of small protruding portions 813e are formed on a vessel outer surface of the gas permeable film, and when the outer surface is brought in contact with a planar surface, a space enabling ventilation is formed between the gas permeable film and the planar surface by the small protruding portions 813e.

Thus, according to the cell culture vessel, the method for producing cells, and the method for producing a cell culture vessel of the embodiment, the cell culture vessel in which the projecting portions are formed without intervals in a mountain range pattern on the inner side of the vessel can be produced, and the cell culture vessel is excellent in gas permeation performance and strength. Using the cell culture vessel enables efficiently culturing cells in a large amount.

### [Ninth Embodiment]

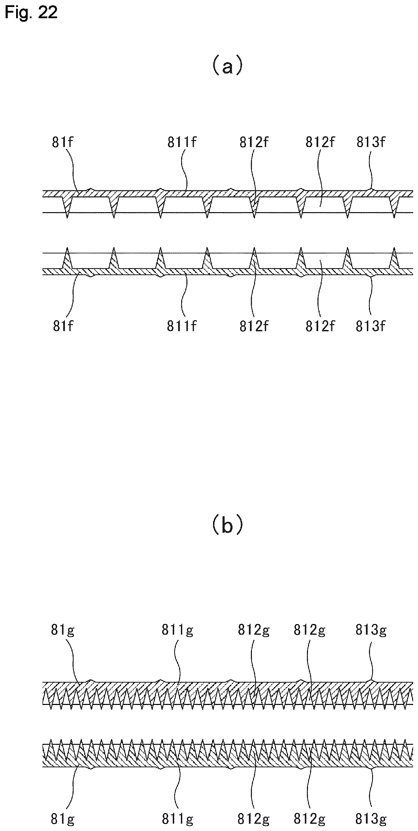

Next, the ninth embodiment of the present invention will be described with reference to FIG. 22(a).

A cell culture vessel of the embodiment is different from the seventh embodiment in that a plurality of thin portions 811f and projecting portions 812f are formed on the gas permeable film, and as the projecting portions 812f, a plurality of approximately triangular prisms are disposed in a grid pattern on a vessel inner surface of the gas permeable film, and the projecting portions 812f mutually adjacent in parallel are formed with intervals.

Other configurations of the cell culture vessel, the method for producing cells, and the method for producing a cell culture vessel of the embodiment are similar to those in the seventh embodiment excluding the points described below.

Specifically, as illustrated in FIG. 22(a), in the cell culture vessel of the embodiment, a plurality of thin portions 811f are formed on a planar substrate 81f as the gas permeable film. Projecting portions 812f are formed in parallel with each other with intervals in a front-back direction (in the drawing, depth direction) and a right-left direction on a vessel inner surface of the gas permeable film. Furthermore, a plurality of small protruding portions 813f are formed on a vessel outer surface of the gas permeable film.

While both of the mutually opposed planar substrates 81f are formed of the gas permeable film, and the thin portions 811f, the projecting portions 812f, and the small protruding portions 813f are formed on each of the gas permeable films in this embodiment, a configuration in which only any one of the planar substrates 81f is formed of the gas permeable film, and the thin portions 811f, the projecting portions 812f, and the small protruding portions 813f are formed on the gas permeable film may be employed.

The projecting portions 812f are preferably formed by arranging a plurality of approximately triangular prisms in parallel in a mountain range pattern and arranging a plurality of other approximately triangular prisms in parallel in a mountain range pattern so as to intersect with the plurality of approximately triangular prisms (grid pattern).

In FIG. 22(a), the heights of the approximately triangular prisms in the horizontal direction are formed to be lower than the heights of the approximately triangular prisms in the vertical direction (in the drawing, depth direction). Accordingly, when the cell culture vessel of the embodiment is placed on a planar surface, a space enabling ventilation is formed between the planar substrate 81f and the planar surface.

By thus forming the projecting portions 812f in a grid pattern, the contacted area of the gas permeable film with the planar surface can be reduced, and the inhibition of the gas permeation performance of the cell culture vessel can be avoided.

With the grid pattern, strengths of the protrusions of the projecting portions 812f can be enhanced.

Further, the projecting portions 812f may be formed such that the plurality of approximately triangular prisms obliquely intersect with the plurality of other approximately triangular prisms.

While the thin portion 811f is formed so as to have a width of 1 mm to 5 mm in the embodiment, the width of the thin portion 811f is preferably 1 mm to 3 mm, and more preferably 1 mm to 2 mm.

In the method for producing a cell culture vessel of the embodiment, it is preferred that as the projecting portions 812f, a plurality of approximately triangular prisms are formed to be arranged in parallel with each other with intervals in a grid pattern on a vessel inner surface of the gas permeable film, a plurality of small protruding portions 813f are formed on a vessel outer surface of the gas permeable film, and when the outer surface is brought in contact with a planar surface, a space enabling ventilation is formed between the gas permeable film and the planar surface by the small protruding portions 813f

Thus, according to the cell culture vessel, the method for producing cells, and the method for producing a cell culture vessel of the embodiment, the cell culture vessel in which the projecting portions are formed with intervals in a grid pattern on the inner side of the vessel can be produced, and the cell culture vessel is excellent in gas permeation performance and strength. Using the cell culture vessel enables efficiently culturing cells in a large amount.

### [Tenth Embodiment]

Next, the tenth embodiment of the present invention will be described with reference to FIG. 22(b).

A cell culture vessel of the embodiment is different from the seventh embodiment in that a plurality of thin portions 811g and projecting portions 812g are formed on the gas permeable film, and as the projecting portions 812g, a plurality of approximately triangular prisms are disposed in a grid pattern on a vessel inner surface of the gas permeable film, and the projecting portions 812g mutually adjacent in parallel are continuously formed without intervals.

Other configurations of the cell culture vessel, the method for producing cells, and the method for producing a cell culture vessel of the embodiment are similar to those in the seventh embodiment excluding the points described below.

Specifically, as illustrated in FIG. 22(b), in the cell culture vessel of the embodiment, a plurality of thin portions 811g are formed in dot shapes on a planar substrate 81g as the gas permeable film. Projecting portions 812g are continuously formed to be parallel without intervals in a front-back direction (in the drawing, depth direction) and a right-left direction on a vessel inner surface of the gas permeable film. Furthermore, a plurality of small protruding portions 813g are formed on a vessel outer surface of the gas permeable film.

While both of the mutually opposed planar substrates 81g are formed of the gas permeable film, and the thin portions 811g, the projecting portions 812g, and the small protruding portions 813g are formed on each of the gas permeable films in this embodiment, a configuration in which only any one of the planar substrates 81g is formed of the gas permeable film, and the thin portions 811g, the projecting portions 812g, and the small protruding portions 813g are formed on the gas permeable film may be employed.

The projecting portions 812g are preferably formed by arranging a plurality of approximately triangular prisms in parallel in a mountain range pattern and arranging a plurality of other approximately triangular prisms in parallel in a mountain range pattern so as to intersect with the plurality of approximately triangular prisms (grid pattern).

In FIG. 22(b), the heights of the approximately triangular prisms in the horizontal direction are formed to be lower than the heights of the approximately triangular prisms in the vertical direction (in the drawing, depth direction). Accordingly, when the cell culture vessel of the embodiment is placed on a planar surface, a space enabling ventilation is formed between the planar substrate 81g and the planar surface.

By thus forming the projecting portions 812g in a grid pattern, the contacted area of the gas permeable film with the planar surface can be reduced, and the inhibition of the gas permeation performance of the cell culture vessel can be avoided.

With the grid pattern, strengths of the protrusions of the projecting portions 812g can be enhanced.

Further, the projecting portions 812g may be formed such that the plurality of approximately triangular prisms obliquely intersect with the plurality of other approximately triangular prisms.

In the method for producing a cell culture vessel of the embodiment, it is preferred that as the projecting portions 812g, a plurality of approximately triangular prisms are formed to be arranged in parallel without intervals in a grid pattern on a vessel inner surface of the gas permeable film, a plurality of small protruding portions 813g are formed on a vessel outer surface of the gas permeable film, and when the outer surface is brought in contact with a planar surface, a space enabling ventilation is formed between the gas permeable film and the planar surface by the small protruding portions 813g.

Thus, according to the cell culture vessel, the method for producing cells, and the method for producing a cell culture vessel of the embodiment, the cell culture vessel in which the projecting portions are continuously formed without intervals in a grid pattern on the inner side of the vessel can be produced, and the cell culture vessel is excellent in gas permeation performance and strength. Using the cell culture vessel enables efficiently culturing cells in a large amount.

### EXAMPLES

First, tests performed for confirming effects of the cell culture vessels according to the embodiments of the present invention will be described.

### [Test 1]

The cell culture vessel of the first embodiment and a conventional cell culture vessel were prepared, and a test for comparing respective gas permeation performances was conducted.

Specifically, as the cell culture vessel of the first embodiment (Example 1), one including protruding portions on both surfaces was prepared. That is, one in which protruding portions having a height of 0.2 mm and a width of 0.2 mm were formed at pitches of 0.42 mm by thermal transfer on the whole of one surface of a film having a thickness of 90 µm formed of a linear low density polyethylene (manufactured by Toyo Seikan Group Holdings, Ltd.) was prepared. At this time, as the protruding portions, a plurality of approximately triangular prisms were formed to be arranged in parallel in a mountain range pattern. The other surface of the film was flat. Then, the two films were stacked so as to have the protruding portions in outer sides, one port was interposed therebetween, and peripheral edge portions were thermally welded, thus forming a bag-shaped one. An outer shape size was 120 mm × 65 mm, and a bottom surface area of a culture space was 48 cm².

As a conventional cell culture vessel (Comparative Example 1), one in which both surfaces were flat without protruding portions on a surface of a film having a thickness of 90 µm formed of a linear low density polyethylene (manufactured by Toyo Seikan Group Holdings, Ltd.) was prepared. Then, one port was interposed between the two films, and peripheral edge portions were thermally welded, thus forming a bag-shaped one. An outer shape size was 120 mm × 65 mm, and a bottom surface area of a culture space was 48 cm².

Next, a non-contact oximeter was installed in each of the cell culture vessels, and 20 ml of pure water was filled. The liquid thickness was 4 mm in each of the vessels. Then, the change of a dissolved oxygen level of water near the bottom surface in each of the cell culture vessels was measured.

At this time, each of the cell culture vessels was secured to a culture jig, and put in a CO₂ incubator at 37°C and left once, thereby making the dissolved oxygen in the vessel about 21% as an oxygen concentration in the atmosphere. After the oxygen concentration in the vessel stabilized, the oxygen concentration in the CO₂ incubator was decreased to 5%.

As the culture jig, one in which the whole of an upper surface of a stage on which the cell culture vessel was placed and the whole of a lower surface of a pressing member pressing the cell culture vessel from the upper side were flat was used. The cell culture vessel is smaller than the upper surface of the stage and the lower surface of the pressing member in size. The same applies to the tests below.

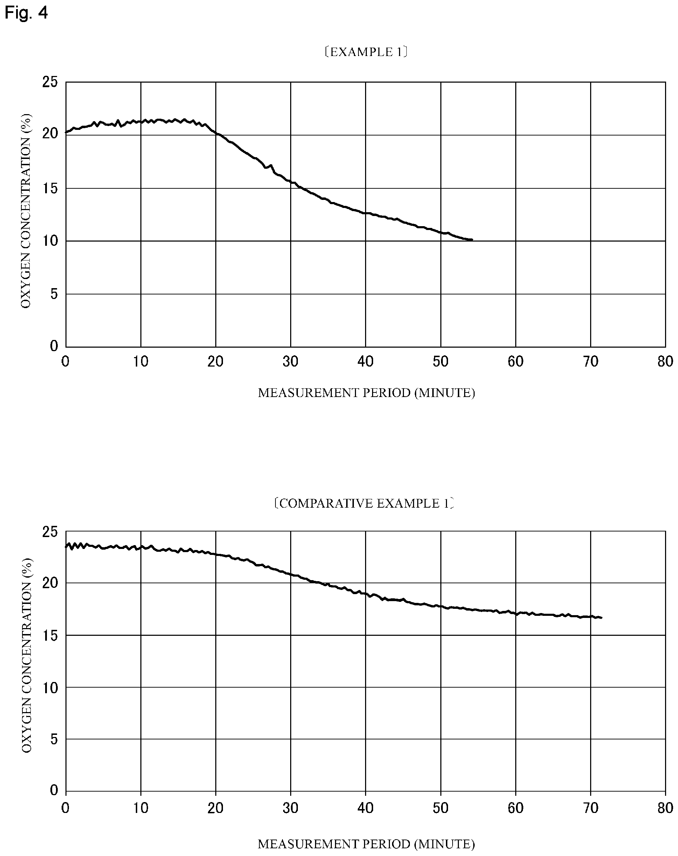

Here, since a difference in oxygen partial pressure is caused between the inside and outside of the vessel due to the change of the oxygen concentration around the vessel from 21% to 5%, the oxygen inside the vessel escapes from the vessel through the gas permeable film. At this time, the change rate of the oxygen concentration is correlated with the gas permeation performance of the film, thus allowing the quantitative measurement of the gas permeation performance. FIG. 4 illustrates the result.

In FIG. 4, the reason why the oxygen concentration in the cell culture vessel was about 24% in Comparative Example 1 while the oxygen concentration in the cell culture vessel was about 20% in Example 1 at the beginning of the measurement is that: as a characteristic of the measurement with the non-contact oximeter used in the test, the change of the element installed in the vessel is contactlessly measured by a measuring device, an accurate value is obtained when the element is in contact with a measurement unit, and the value increases as the element is spaced more from the measurement section, and therefore, the dissolved oxygen has a large value depending on the thickness or the installation state of the vessel while the dissolved oxygen is usually 21% at 37°C. However, since the change amount is not affected, and the oxygen permeability is determined based on the change amount, the measurement has no problem on the performance evaluation.

As illustrated in FIG. 4, the maximum value of the oxygen concentration change amount per minute was 0.6 %/minute in the cell culture vessel of Example 1. In contrast, the maximum value of the oxygen concentration change amount per minute was 0.2 %/minute in the cell culture vessel of Comparative Example 1. That is, it was seen that the gas permeation performance of Example 1 was three times greater than that of Comparative Example 1, and the cell culture vessel of the embodiment had an excellent oxygen permeability.

### [Test 2]

Cells were cultured using the cell culture vessel of the first embodiment and a conventional cell culture vessel, and a test for comparing respective culture performances was conducted.

Specifically, as the cell culture vessel of the first embodiment (Example 2), one including protruding portions on both surfaces was prepared. That is, one in which protruding portions having a height of 0.2 mm and a width of 0.2 mm were formed at pitches of 0.42 mm by thermal transfer on the whole surface of a film having a thickness of 90 µm formed of a linear low density polyethylene (LLDPE, manufactured by Toyo Seikan Group Holdings, Ltd.) was prepared. At this time, as the protruding portions, a plurality of approximately triangular prisms were formed to be arranged in parallel in a mountain range pattern. The other surface of the film was flat. Then, the two films were stacked so as to have the protruding portions in outer sides, two ports were interposed therebetween at both ends in a longitudinal direction, and peripheral edge portions were thermally welded, thus forming a bag-shaped one. An outer shape size was 120 mm × 65 mm, and a bottom surface area of a culture space was 48 cm².

As a conventional cell culture vessel (Comparative Example 2), one in which both surfaces were flat without protruding portions on a surface of a film having a thickness of 90 µm formed of a linear low density polyethylene (manufactured by Toyo Seikan Group Holdings, Ltd.) was prepared. Then, two ports were interposed between the two films at both ends in the longitudinal direction, and peripheral edge portions were thermally welded, thus forming a bag-shaped one. An outer shape size was 120 mm × 65 mm, and a bottom surface area of a culture space was 48 cm².

Furthermore, as the culture jig, one in which the whole of an upper surface of a stage on which the cell culture vessel was placed and the whole of a lower surface of a pressing member pressing the cell culture vessel from the upper side were flat was used.

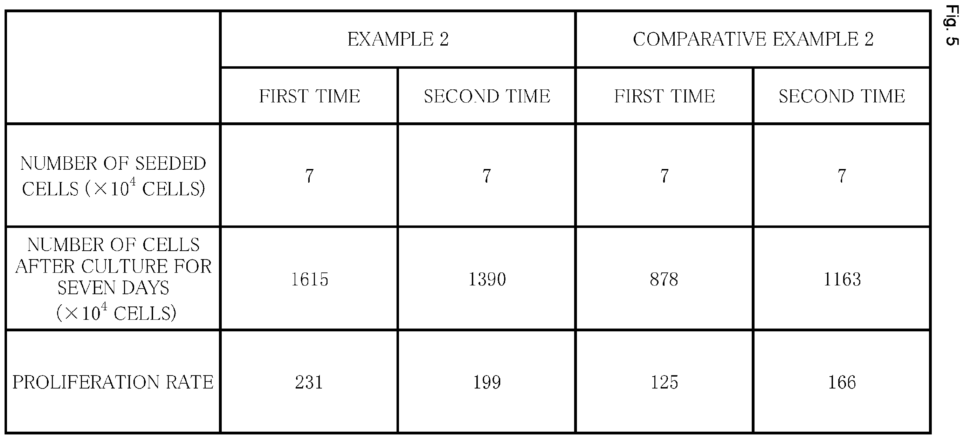

As cells to be cultured, iPS cells (cell line 1231A3) were used. As the culture fluid, StemFit AK02N (Ajinomoto Co., Inc.) was used, and they were filled into each of the cell culture vessels. The filled culture fluid was 10 ml at 37°C, and the liquid thickness was 2 mm in each of the vessels. At this time, 10 µM of Y-27632 and 24 µl of an adhesive substrate (i-Matrix, Laminin 511-E8) were added to 10 ml of the culture fluid in the cell culture vessel, 70,000 cells were injected, and the cell culture vessel was set to the cell culture jig, thus starting the culture. One, four, five, and six days after seeding the cells, 10 ml of the culture fluid (only the culture fluid to which Y-27632 and the adhesive substrate were not added) was entirely changed, and the culture was performed for seven days. Seven days after, the cells were recovered using a dissociation liquid TrypLE, and the number of cells was measured, thus calculating a proliferation rate. These processes were performed twice for each cell culture vessel. FIG. 5 illustrates the result.

As illustrated in the drawing, the proliferation rate of the cells by the cell culture vessel of Example 2 exhibits the more excellent result than that of Comparative Example 2, and it was seen that the cell culture vessel of the embodiment provided the appropriate gas permeation performance.

Next, a test performed for confirming the effect of the cell culture jig according to the embodiment of the present invention will be described.

### [Test 3]

The cell culture jig of the second embodiment and a conventional cell culture jig were prepared, and the same cell culture vessel was secured to each of the cell culture jigs, thus performing the test for comparing the gas permeation performances.

Specifically, as the cell culture jig of the second embodiment (Example 3), one in which protruding portions were formed on the whole of an upper surface of a stage on which the cell culture vessel was placed and the whole of a lower surface of a pressing member pressing the cell culture vessel from the upper side was prepared.

Polycarbonate was used as materials of the stage and the pressing member, the mountain range patterns as included in the above-described embodiments were formed on the whole upper surface of the stage and the whole lower surface of the pressing member as the protruding portions. At this time, for the protrusion in the formed mountain range pattern, the height was 200 µm, the width was 400 µm, and the pitch was 800 µm. The sizes of the whole upper surface of the stage and the whole lower surface of the pressing member are larger than the size of the cell culture vessel secured thereto described below. The same applies to the tests below.

As a substrate of a cell culture vessel secured to the cell culture jig, a film having both flat surfaces was prepared. The film had a thickness of 110 µm, and was formed of a linear low density polyethylene (manufactured by Toyo Seikan Group Holdings, Ltd.). Then, one port was interposed between the two films, and peripheral edge portions were thermally welded, thus forming a bag-shaped one. An outer shape size was 120 mm × 65 mm, and a bottom surface area of a culture space was 48 cm².

As a conventional cell culture jig (Comparative Example 3), one in which the whole of an upper surface of a stage on which the cell culture vessel was placed and the whole of a lower surface of a pressing member pressing the cell culture vessel from the upper side were flat was prepared.

As materials of the stage and the pressing member, plane plates formed of polycarbonate were used.

Furthermore, as a cell culture vessel secured to the cell culture jig, the same one as Example 3 was prepared.

Next, a non-contact oximeter was installed in each of the cell culture vessels, and 20 ml of pure water was filled. The liquid thickness was 4 mm in each of the vessels. Then, the change of a dissolved oxygen level of water near the bottom surface in each of the cell culture vessels was measured.

At this time, the cell culture vessels were secured to the respective cell culture jigs, and put in a CO₂ incubator at 37°C and left once, thereby making the dissolved oxygen in the vessel about 21% as an oxygen concentration in the atmosphere. After the oxygen concentration in the vessel stabilized, the oxygen concentration in the CO₂ incubator was decreased to 10%, and then, the oxygen concentration in the CO₂ incubator was increased to 21%.

Here, since a difference in oxygen partial pressure is caused between the inside and outside of the vessel due to the change of the oxygen concentration around the vessel from 21% to 10%, the oxygen inside the vessel escapes from the vessel through the gas permeable film. Additionally, due to the change of the oxygen concentration around the vessel from 10% to 21%, the oxygen outside the vessel enters the vessel through the gas permeable film. At this time, the change rate of the oxygen concentration is correlated with the gas permeation performance of the film, thus allowing the quantitative measurement of the gas permeation performance. FIG. 11 illustrates the result.

In FIG. 11, the reason why the oxygen concentration in the cell culture vessel of Comparative Example 3 was decreased only to about 12% while the oxygen concentration in the cell culture vessel of Example 3 was decreased to about 10% was that: since the oxygen concentration decrease rate of Comparative Example 3 was low and it was expected to take a considerable time to decrease the oxygen concentration to 10% similarly to Example 3, the measurement was performed by halting the decrease of the oxygen concentration at about 12% and increasing back the oxygen concentration to 21% in Comparative Example 3.

As illustrated in FIG. 11, when the oxygen concentration around the vessel was changed from 21% to 10%, the maximum value of the oxygen concentration change amount per minute was 0.75 %/minute in the cell culture vessel of Example 3. In contrast, the maximum value of the oxygen concentration change amount per minute was 0.25 %/minute in the cell culture vessel of Comparative Example 3. When the oxygen concentration around the vessel was changed from 10% to 21%, the maximum value of the oxygen concentration change amount per minute was 0.6 %/minute in the cell culture vessel of Example 3. In contrast, the maximum value of the oxygen concentration change amount per minute was 0.3 %/minute in the cell culture vessel of Comparative Example 3.

That is, when the oxygen concentration around the vessel was changed from 21% to 10%, the gas permeation performance of Example 3 was about three times greater than that of Comparative Example 3, and when the oxygen concentration around the vessel was changed from 10% to 21%, the gas permeation performance of Example 3 was about two times greater than that of Comparative Example 3.

Thus, it was seen that the cell culture jig of the embodiment was excellent in oxygen permeation performance compared with the conventional cell culture jig.

### [Test 4]

Cell culture jigs having different configurations (Example 4, Example 5) were prepared as the cell culture jig of the second embodiment, and the same cell culture vessel was secured to each of the cell culture jigs, thus performing the test for comparing the gas permeation performances.

Specifically, as the cell culture jig of the second embodiment (Example 4), one in which protruding portions were formed on the whole of a lower surface of a pressing member pressing the cell culture vessel from the upper side was prepared. The protruding portions were not formed on an upper surface of a stage on which the cell culture vessel was placed.

Polycarbonate was used as a material of the pressing member, and the mountain range pattern was formed similarly to Example 3 on the whole lower surface of the pressing member as the protruding portions. As a material of the stage, a plane plate formed of polycarbonate was used.

As a cell culture vessel secured to the cell culture jig, the same one as Example 3 was prepared.

Next, as the cell culture jig of the second embodiment (Example 5), one in which protruding portions were formed on the whole of an upper surface of a stage on which the cell culture vessel was placed was prepared. The protruding portions were not formed on a lower surface of a pressing member pressing the cell culture vessel from the upper side.

Polycarbonate was used as a material of the stage, and the mountain range pattern was formed similarly to Example 3 on the whole upper surface of the stage as the protruding portions. As a material of the pressing member, a plane plate formed of polycarbonate was used.

As a cell culture vessel secured to the cell culture jig, the same one as Example 3 was prepared.

Next, a non-contact oximeter was installed in each of the cell culture vessels, and 20 ml of pure water was filled. The liquid thickness was 4 mm in each of the vessels. Then, the change of a dissolved oxygen level of water near the bottom surface in each of the cell culture vessels was measured.

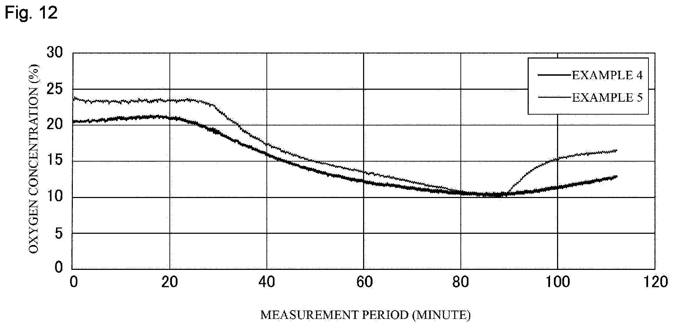

At this time, the cell culture vessels were secured to the respective cell culture jigs, and put in a CO₂ incubator at 37°C and left once, thereby making the dissolved oxygen in the vessel about 21% as an oxygen concentration in the atmosphere. After the oxygen concentration in the vessel stabilized, the oxygen concentration in the CO₂ incubator was decreased to 10%, and then, the oxygen concentration in the CO₂ incubator was increased to 21%. FIG. 12 illustrates the result.

In FIG. 12, the reason why the oxygen concentration in the cell culture vessel was about 24% in Example 5 while the oxygen concentration in the cell culture vessel was about 20% in Example 4 at the beginning of the measurement is that: as a characteristic of the measurement with the non-contact oximeter used in the test, the change of the element installed in the vessel is contactlessly measured by a measuring device, an accurate value is obtained when the element is in contact with a measurement unit, and the value increases as the element is spaced more from the measurement section, and therefore, the dissolved oxygen has a large value depending on the thickness or the installation state of the vessel while the dissolved oxygen is usually 21% at 37°C. However, since the change amount is not affected, and the oxygen permeation performance is determined based on the change amount, the measurement has no problem on the performance evaluation.

As illustrated in FIG. 12, when the oxygen concentration around the vessel was changed from 21% to 10%, the maximum value of the oxygen concentration change amount per minute was 0.4 %/minute in the cell culture vessel in Example 4. In contrast, the maximum value of the oxygen concentration change amount per minute was 0.8 %/minute in the cell culture vessel of Example 5. When the oxygen concentration around the vessel was changed from 10% to 21%, the maximum value of the oxygen concentration change amount per minute was 0.25 %/minute in the cell culture vessel of Example 4. In contrast, the maximum value of the oxygen concentration change amount per minute was 1.1 %/minute in the cell culture vessel of Example 5.

That is, when the oxygen concentration around the vessel was changed from 21% to 10%, the gas permeation performance of Example 5 was about two times greater than that of Example 4, and when the oxygen concentration around the vessel was changed from 10% to 21%, the gas permeation performance of Example 5 was about four times greater than that of Example 4.

Thus, it was seen that the effect of improving the oxygen permeability of the cell culture vessel used for the cell culture in a manner of being secured to the cell culture jig was more enhanced in the case where the protruding portions of the cell culture jig were formed on the upper surface of the stage on which the cell culture vessel was placed than in the case where the protruding portions of the cell culture jig were formed on the lower surface of the pressing member pressing the cell culture vessel from the upper side.

### [Test 5]

The cell culture jig of the second embodiment and a conventional cell culture jig were prepared, the same cell culture vessel was secured to each of the cell culture jigs, and cells were cultured, thus performing the test for comparing the culture performances of them.

Specifically, as the cell culture jig of the second embodiment (Example 6), the same one as Example 3 was prepared. That is, one in which protruding portions were formed on the whole of an upper surface of a stage on which the cell culture vessel was placed and the whole of a lower surface of a pressing member pressing the cell culture vessel from the upper side was prepared.

The protruding portions were formed on the whole upper surface of the stage and the whole lower surface of the pressing member in the mountain range pattern.

As a cell culture vessel secured to the cell culture jig, the same one as Example 3 was prepared.

As a conventional cell culture jig (Comparative Example 4), the same one as Comparative Example 3 was prepared. That is, one in which the whole of an upper surface of a stage on which the cell culture vessel was placed and the whole of a lower surface of a pressing member pressing the cell culture vessel from the upper side were flat was prepared.

Furthermore, as a cell culture vessel secured to the cell culture jig, the same one as Example 3 was prepared.

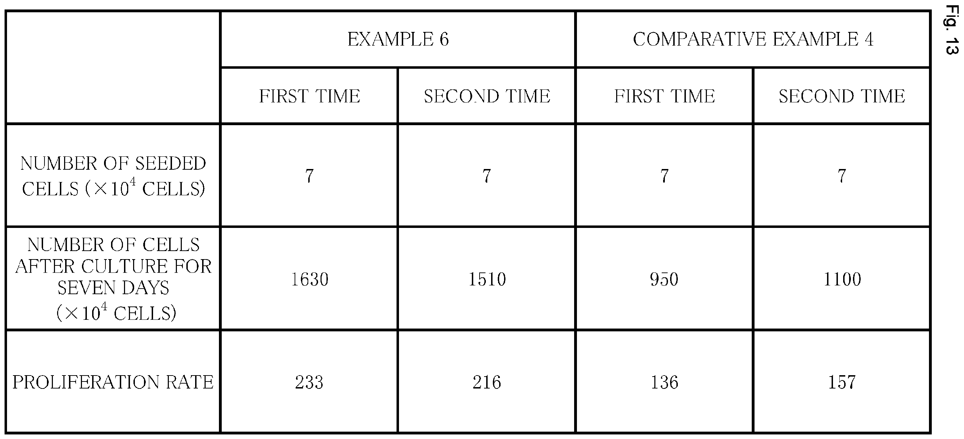

As cells to be cultured, iPS cells (cell line 1231A3) were used. As the culture fluid, StemFit AK02N (Ajinomoto Co., Inc.) was used, and they were filled into each of the cell culture vessels. The filled culture fluid was 10 ml at 37°C, and the liquid thickness was 2 mm in each of the vessels. At this time, 10 µM of Y-27632 and 24 µl of an adhesive substrate (i-Matrix, Laminin 511-E8) were added to 10 ml of the culture fluid in the cell culture vessel, 70,000 cells were injected, and the cell culture vessel was set to the cell culture jig, thus starting the culture. One, four, five, and six days after seeding the cells, 10 ml of the culture fluid (only the culture fluid to which Y-27632 and the adhesive substrate were not added) was entirely changed, and the culture was performed for seven days. Seven days after, the cells were recovered using a dissociation liquid TrypLE, and the number of cells was measured, thus calculating a proliferation rate. These processes were performed twice for each cell culture jig. FIG. 13 illustrates the result.

As illustrated in the drawing, the proliferation rate of the cells by the cell culture vessel exhibits the more excellent result in Example 6 than Comparative Example 4, and it was seen that the inhibition of the gas permeation performance of the cell culture vessel was suppressed and cells were able to be cultured with a high density according to the cell culture jig of the embodiment.

### [Test 6]

The cell culture jig of the second embodiment and a conventional cell culture jig were prepared, the same cell culture vessel was secured to each of the cell culture jigs, and cells were cultured, thus performing the test for comparing the culture performances of them.

Specifically, as the cell culture jig of the second embodiment (Example 7), one in which the whole of an upper surface of a stage on which the cell culture vessel was placed was formed of a porous material was prepared.

As a lower surface of a pressing member pressing the cell culture vessel from the upper side, one formed of a porous material was not used, but flat one was prepared. As a material of the stage, a porous filter plate made of polypropylene (hole diameter 400 µm, thickness 3 mm, AS ONE Corporation. 3-2531-02) was used. As a material of the pressing member, a plane plate made of polycarbonate was used.

As a cell culture vessel secured to the cell culture jig, the same one as Example 3 was prepared.

As a conventional cell culture jig (Comparative Example 5), the same one as Comparative Example 3 was prepared. That is, one in which the whole of an upper surface of a stage on which the cell culture vessel was placed and the whole of a lower surface of a pressing member pressing the cell culture vessel from the upper side were flat was prepared.

Furthermore, as a cell culture vessel secured to the cell culture jig, the same one as Example 3 was prepared.

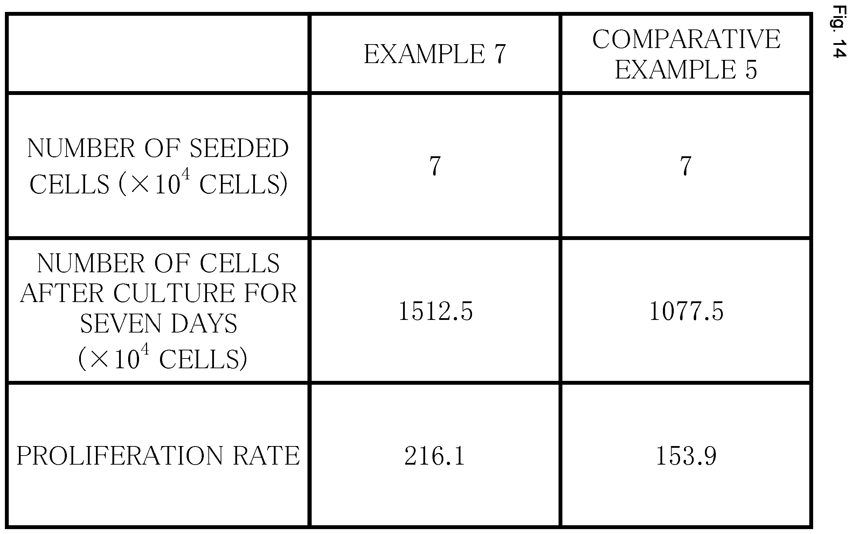

As cells to be cultured, iPS cells (cell line 1231A3) were used. As the culture fluid, StemFit AK02N (Ajinomoto Co., Inc.) was used, and they were filled into each of the cell culture vessels. The filled culture fluid was 10 ml at 37°C, and the liquid thickness was 2 mm in each of the vessels. At this time, 10 µM of Y-27632 and 24 µl of an adhesive substrate (i-Matrix, Laminin 511-E8) were added to 10 ml of the culture fluid in the cell culture vessel, 70,000 cells were injected, and the cell culture vessel was set to the cell culture jig, thus starting the culture. One, four, five, and six days after seeding the cells, 10 ml of the culture fluid (only the culture fluid to which Y-27632 and the adhesive substrate were not added) was entirely changed, and the culture was performed for seven days. Seven days after, the cells were recovered using a dissociation liquid TrypLE, and the number of cells was measured, thus calculating a proliferation rate. FIG. 14 illustrates the result.

As illustrated in the drawing, the proliferation rate of the cells by the cell culture jig exhibits the more excellent result in Example 7 than Comparative Example 5, and it was seen that the inhibition of the gas permeation performance of the cell culture vessel was suppressed and cells were able to be cultured with a high density according to the cell culture jig of the embodiment.

The present invention is not limited to the above-described embodiments and examples, and, needless to say, various modifications can be made within the scope of the present invention. The present invention can be appropriately changed, for example, the shape of the protruding portion or the projecting portion formed on the cell culture vessel or the cell culture jig is changed to various shapes different from those in the embodiments, or the third embodiment to the tenth embodiment are partially combined to configure a cell culture vessel in which the projecting portions are formed on the outer side and the inner side of the vessel.

### INDUSTRIAL APPLICABILITY

The present invention can be appropriately used, for example, when cells are cultured in a large amount with a high density using a cell culture bag.

The entire contents of documents mentioned in this specification and the specification of the Japanese patent application to which this application claims priority under the Paris Convention are entirely incorporated by reference herein.

### DESCRIPTION OF REFERENCE SIGNS

- 1: 1 Cell culture vessel
- 11: Planar substrate
- 111: Protruding portion
- 12: Port
- 2: Cell
- 3: Cell culture apparatus
- 31: Stage
- 32: Pressing member
- 33: Guide pin
- S: Culture space
- H: Peripheral edge portion
- V: Ventilation space
- 5, 5a: Cell culture jig
- 51, 51a: Stage
- 511, 511a: Protruding portion
- 52, 52a: Pressing member
- 521, 521a: Protruding portion
- 53: Support pillar
- 54: Top panel
- 55, 55a: Guide pin
- 6: Cell culture vessel
- 61, 62: Planar substrate
- 63: Port
- 8: Cell culture vessel
- 81, 81a to 81g: Planar substrate
- 811, 811a to 811g: Thin portion
- 812, 812a to 812g: Projecting portion
- 813d to 813g: Small protruding portion
- 82: Port
- 9: Cell

## Claims

1. A bag-shaped cell culture vessel of a closed system, comprising:
at least one port; and
mutually opposed planar substrates, wherein
at least one of the planar substrates is formed of a gas permeable film, the at least one gas permeable film has an outer surface provided with a protruding portion having a shape different from an inner surface shape of the gas permeable film, a culture space for culturing cells is provided in the inner surface side of the gas permeable film, and when the gas permeable film is brought in contact with a planar surface, a space enabling ventilation is formed between the gas permeable film and the planar surface by the protruding portion.

2. The cell culture vessel according to claim 1, wherein
in an outer surface region of the gas permeable film in an outer side of the culture space, a plurality of approximately triangular prisms are formed to be arranged in parallel in a mountain range pattern as the protruding portion.

3. The cell culture vessel according to claim 1 or 2, wherein
both of the mutually opposed planar substrates are formed of a gas permeable film, and the protruding portions are disposed on outer surfaces of the respective gas permeable films.

4. The cell culture vessel according to any one of claims 1 to 3, wherein
the inner surface of the gas permeable film is flat without unevenness.

5. The cell culture vessel according to any one of claims 1 to 3, wherein
the inner surface of the gas permeable film is provided with unevenness.

6. The cell culture vessel according to any one of claims 1 to 5, wherein
a region without the protruding portion of the gas permeable film has a thickness of less than 100 µm.

7. The cell culture vessel according to any one of claims 1 to 5, wherein
a region without the protruding portion of the gas permeable film has a thickness of from 70 µm to 10 µm.

8. A method for producing a bag-shaped cell culture vessel of a closed system, the cell culture vessel including at least one port and mutually opposed planar substrates, the method comprising:
using a gas permeable film for at least one of the planar substrates, and forming a protruding portion having a shape different from an inner surface shape of the gas permeable film on an outer surface of the gas permeable film such that a space enabling ventilation is formed between the gas permeable film and a planar surface when the gas permeable film is brought in contact with the planar surface; and
forming a culture space for culturing cells in the inner surface side of the gas permeable film by bonding peripheral edge portions of the mutually opposed planar substrates.

9. The method for producing a cell culture vessel according to claim 8, wherein
the gas permeable film is used for both of the mutually opposed planar substrates, and the protruding portions are formed on outer surfaces of the respective gas permeable films.

10. The method for producing a cell culture vessel according to claim 8 or 9, wherein
the protruding portion is formed by a thermal transfer, a cast film forming, or an inflation film forming.

11. A method for producing cells in which cells are cultured using the cell culture vessel according to any one of claims 1 to 7.

12. A cell culture apparatus comprising:
the cell culture vessel according to any one of claims 1 to 7; and
a culture jig that includes a flat stage on which the cell culture vessel is placed and a pressing member that presses the cell culture vessel placed on the stage, the culture jig being provided with a space enabling ventilation between the gas permeable film and the stage and/or between the gas permeable film and the pressing member by the protruding portion formed on the cell culture vessel.

13. A cell culture jig for holding a bag-shaped cell culture vessel of a closed system, the cell culture vessel including mutually opposed planar substrates, at least one of the planar substrates being formed of a gas permeable film, the cell culture jig comprising:
a stage on which the cell culture vessel is placed; and
a pressing member that presses the cell culture vessel to the stage, wherein
the stage is formed of a substrate enabling ventilation between the gas permeable film and the stage, and the gas permeable film side of the cell culture vessel is placed on the stage.

14. The cell culture jig according to claim 13, wherein
the pressing member is formed of a substrate enabling ventilation between the gas permeable film and the pressing member, and the gas permeable film side of the cell culture vessel is pressed by the pressing member.

15. The cell culture jig according to claim 13 or 14, wherein
guide pins are disposed upright on four corners of the stage, the guide pins pass through guide bores provided to the pressing member, and the pressing member is disposed to be movable in a perpendicular direction along the guide pins.

16. The cell culture jig according to claim 13 or 14, wherein
support pillars are disposed upright on four corners of the stage, a top panel is secured to the support pillars, the top panel includes guide pins, the guide pins pass through guide bores provided to the pressing member, and the pressing member is disposed to be movable in a perpendicular direction along the guide pins.

17. The cell culture jig according to any one of claims 13 to 16, wherein
the substrate enabling ventilation is a porous material.

18. The cell culture jig according to any one of claims 13 to 16, wherein
the substrate enabling ventilation is a perforated plate.

19. The cell culture jig according to any one of claims 13 to 16, wherein
the substrate enabling ventilation is formed by uneven processing of a surface of the substrate.

20. The cell culture jig according to claim 19, wherein
a protruding portion is formed on the surface of the substrate enabling ventilation by the uneven processing.

21. The cell culture jig according to claim 20, wherein
a plurality of approximately triangular prisms are formed to be arranged in parallel in a mountain range pattern as the protruding portion.

22. A method for producing cells in which cells are cultured using the cell culture jig according to any one of claims 13 to 21.

23. A bag-shaped cell culture vessel of a closed system, comprising:
at least one port; and
mutually opposed planar substrates, wherein
at least one of the planar substrates is formed of a gas permeable film, a culture space for culturing cells is provided in a vessel inner surface side of the gas permeable film, a plurality of thin portions and projecting portions are formed on the gas permeable film forming the culture space, and the thin portion has a thickness of 75 µm or less.

24. The cell culture vessel according to claim 23, wherein
the thin portion has the thickness of 20 µm to 35 µm.

25. The cell culture vessel according to claim 23 or 24, wherein
a surface of the gas permeable film on which the projecting portions are formed is a vessel outer surface of the gas permeable film, and a vessel inner surface of the gas permeable film is flat without unevenness.

26. The cell culture vessel according to claim 23 or 24, wherein
a surface of the gas permeable film on which the projecting portions are formed is a vessel inner surface of the gas permeable film, a plurality of small protruding portions are formed on a vessel outer surface of the gas permeable film, and a space enabling ventilation is formed between the gas permeable film and a planar surface by the small protruding portions when the outer surface is brought in contact with the planar surface.

27. The cell culture vessel according to any one of claims 23 to 26, wherein
both of the mutually opposed planar substrates are formed of the gas permeable film, and the plurality of thin portions and the projecting portions are formed on each of the gas permeable films.

28. The cell culture vessel according to any one of claims 23 to 27, wherein
a plurality of approximately triangular prisms are formed to be arranged in parallel in a mountain range pattern as the projecting portions.

29. The cell culture vessel according to any one of claims 23 to 27, wherein
a plurality of approximately triangular prisms are formed to be arranged in parallel in a mountain range pattern as the projecting portions, a plurality of other approximately triangular prisms are formed to be arranged in parallel in a mountain range pattern so as to intersect with the plurality of approximately triangular prisms, and the plurality of approximately triangular prisms are formed in a grid pattern.

30. The cell culture vessel according to any one of claims 23 to 29, wherein
the projecting portions mutually adjacent in parallel are formed with intervals of 1 mm to 5 mm.

31. The cell culture vessel according to any one of claims 23 to 29, wherein
the projecting portions mutually adjacent in parallel are formed without intervals, and the thin portions are formed in line shapes or dot shapes.

32. A method for producing cells in which cells are cultured using the cell culture vessel according to any one of claims 23 to 31.

33. A method for producing a bag-shaped cell culture vessel of a closed system, the cell culture vessel including at least one port and mutually opposed planar substrates, the method comprising:
using a gas permeable film for at least one of the planar substrates, and forming a plurality of thin portions having thicknesses of 75 µm or less and projecting portions on the gas permeable film; and
forming a culture space for culturing cells in a vessel inner surface side of the gas permeable film by bonding peripheral edge portions of the mutually opposed planar substrates.
